# EUROPEAN PATENT APPLICATION

(11) **EP 4 730 368 A2**
(43) Date of publication of application: **22.04.2026**
(21) Application number: 26154646.9
(22) Date of filing: 16.08.2024
(51) Int. Cl.: H01F 5/00

(54) **ELECTRIC COMPONENTS INCLUDING COILS AND METHODS TO FABRICATE THE SAME BY 3D PRINTING**

(30) Priority: 17.08.2023 US 202363533258 P
(62) Divisional of application: 24194993.2
(71) Applicant: Biosense Webster (Israel) Ltd., 2066717 Yokneam (IL)
(72) Inventor: HAYAT, Nathanel Yehuda David, 2066717 Yokneam (IL); BERKOWITZ, Shemer Shmaryau, 2066717 Yokneam (IL); BREEN, Barry Neal, 2066717 Yokneam (IL); BAR-TAL, Meir, 2066717 Yokneam (IL); ZOHAR, Daniel, 2066717 Yokneam (IL); COHEN, Avraham, 2066717 Yokneam (IL)
(74) Representative: Carpmaels & Ransford LLP

(57) **Abstract**

Electric components including coils and methods to fabricate the same by 3D-printing are disclosed. The method includes: 3D printing a magnetic material to form a magnetic channel comprising a magnetic core of the coil device; 3D printing a conductive material to form a conductive channel, including conductive windings of the coil device with turns surrounding the magnetic core; and 3D printing a non-magnetic electrically insulating material to form electrical insulation between the turns of the conductive windings of the coil device. In some embodiments functional structures of the electric component, including the turns of the conductive windings of the coil and the electrical insulation between the turns, are each printed with minimal in-layer feature size of at least two voxels of the 3D-printing. Some embodiments facilitate 3D-printing of flattened coil devices having low aspect ratio between their lengths along their magnetic axes and their widths perpendicular thereto.

## Description

### CROSS-REFERENCE TO RELATED APPLICATION

This application claims the benefit of U.S. Patent Provisional Application 63/533,258, filed August 17, 2023, the contents of which are incorporated by reference as if set forth in its entirety herein.

### TECHNOLOGICAL FIELD

The present invention is in the field of magnetic field sensors and specifically relates to techniques of fabricating electric components including coils which are suitable for magnetic-field-based position sensing, particularly for sensing the positions of medical devices.

### BACKGROUND

In various therapeutic and diagnostic procedures, a catheter/probe is inserted into a patient's body (e.g., chamber of the heart) and to be brought into contact with a body tissue there. Typically, in such procedures, it is necessary to determine the catheter's location within the body (i.e., the location at which a distal tip of the catheter engages the body tissue) as well as the pressure applied thereby to the tissue.

Catheters having integrated location and pressure sensors for sensing the location of the catheter and the pressure/force applied thereby at the contact region with the tissue, are generally known. Such catheters typically utilize inductive coils for determining the location of the catheter within the body and/or the pressure/force applied thereby to a body tissue it engages with.

Conventional techniques for such catheters often utilize wire-winded coils to which a ferrite core may be manually inserted after the winding. The coils fabricated in this way are then soldered to a cable that carries the coil signal to processing circuits, and fitted within the catheter's body.

### BRIEF DESCRIPTION OF THE DRAWINGS

In order to better understand the subject matter that is disclosed herein and to exemplify how it may be carried out in practice, embodiments will now be described, by way of non-limiting example only, with reference to the accompanying drawings, in which:
**Figs. 1A and 1B** illustrate a 3D-printed electric component **100** including a coil **120,** according to an embodiment of the invention;
**Fig. 1C** illustrates a cross section of the coil in the electric component **100** of **Figs. 1A and 1B****;**
**Fig. 2** is a flow diagram of a method **200** to fabricate, by 3D-printing, of one or more coils **120,** or electric components **100** including the same, according to some embodiments of the present invention;
**Fig. 3A** illustrates a perspective view of the electric component 100 including a coil **120,** according to another embodiment of the present invention;
**Fig. 3B** illustrates a side view of a coil **120** within the electric component **100** , according to yet another embodiment of the present invention;
**Fig. 3C** illustrates a top view of a cross section of the coil **120** within the electric component **100** of **Figs 3A and 3B****;**
**Fig. 3D** illustrates the cross section of the coil **120** shown in **Fig. 1C** with grid lines indicating the distribution of pixels/voxels withing a certain 3D-printed plan/layer of the coil **120;**
**Fig. 3E** illustrates a perspective cross-section view of the conductive windings **124** of the coil **120,** taken along the cross-section plane **CP** shown in **Fig. 3D****;**
**Fig. 3F** **and** **3G** illustrate two respective section cuts of the conductive windings **124** taken along the respective section planes **SP1** and **SP2** shown in **Fig. 3E****;**
**Figs. 3H** and **3I** are two respective side views of the conductive windings **124,** taken respectively from lateral side **A** of the coil **120** and from lateral side **C** of the coil **120** of **Fig. 3A****.**
**Fig. 4A** illustrates a perspective view of a 3D printed electronic component **100** which includes/formed-as a flattened coil **120** having low/small aspect ratio between its length along its magnetic axis and its width, according to yet another embodiment of the present invention;
**Fig. 4B** illustrates a side view of the flattened coil **120** 3D printed in the electronic component **100** of **Fig. 4A****;**
**Figs. 5A, 5B, 5C and 5D** illustrate several views of an electric component, in these examples a coil device **120,** with helical magnetic core, 3D printed according to some embodiments of the present invention;
**Figs. 6A, 6B and 6C** illustrate several views of an electric component, in these examples a coil **120** with a plurality magnetic subchannels, each including a helical shaped magnetic core section, which is 3D printed according to some embodiments of the present invention;
**Figs. 7A and 7B** illustrate several views of an electric component, in these examples a coil **120** with meander-like shaped magnetic core, 3D according to some embodiments of the present invention.
**Figs. 8A and 8B** illustrate several views of an electric component, in these examples **a** coil **120** with coiled magnetic core, 3D according to some embodiments of the present invention.
**Fig. 9** is an illustration of a medical instrument **1000** furnished having a magnetic-field based positioning sensor **300** including coils configured according to an embodiment of the present invention.

Like reference numerals are used in the figures to designate elements/parts of the electric components of the invention which have similar functionalities. For brevity and conciseness, the description of such similar elements/parts is not necessarily repeated with respect to each of the embodiments, yet it should be appreciated that in embodiments where not otherwise indicated, the description of elements/parts having the same configuration and/or functionality made in any one of the embodiments, may also be applicable in other embodiments.

### DETAILED DESCRIPTION OF EMBODIMENTS

There is a need in the art of catheters, for physically agile and sensitive catheters having compact/narrow dimensions, that are capable of providing accurate feedback on the location of the catheter in the body, and the contact interface between the catheter and the tissue and the force (e.g., vector) applied between them.

U.S. patent No. U.S.8,535,308 and U.S. patent application No. U.S. 2020/015693, which are both assigned to the assignee of the present Application, disclose for example configurations of such catheters utilizing sensory circuits having several coils, for sensing the location and orientation of the catheter body, as well as the force applied thereby at the interface with the tissue.

One challenge in the art of such catheters is to fit accurate location and force sensors within the narrow body of the catheter, whose lateral dimensions are typically only several millimeters. Indeed, as indicated above, in conventional catheters technologies, the location and force sensors generally utilize miniature wire-winded coils to which a ferrite core may be manually inserted with an open magnetic circuit configuration in order to yield sufficient induced-voltages suitable for location and force sensing of the catheter components. These conventional techniques are however time consuming and costly and involve delicate manual operations for fitting of the coils within the small dimensions of the catheters body.

Therefore, there is a need in the art for more efficient, cost-effective techniques for fabrication of such catheters and for the fabrication of small high sensitivity coils (i.e., having high induced voltage in response to magnetic fields) suitable for use for location and or force sensing in catheters such as those disclosed above. Moreover, there is a need in the art for automated fabrication techniques of such coils, or of electronic components comprising the same, that is suitable for mass production with yield of standardized coils having small variability between fabricated coils of the same design. Further, in the interest of further advent in the art of catheters, there is also a need in the art to facilitate fabrication of even smaller and/or of higher impedance/induced-voltage, coils so as to improve either or both of the sensitivity and agility, and/or reduced dimensions, of the catheter.

The present invention provides a novel and inventive electric component including one or more coils suitable for use as/in magnetic field sensor of a magnetic-field-based positioning system. Moreover, the present invention provides a novel and inventive method of fabrication of the electric components including such coil(s) utilizing 3D printing technique. In embodiments the electric component may be fabricated with coil(s) of compact dimensions while providing relatively high induced-voltage in response to sensed magnetic fields, and is therefore particularly suited for use as a position sensor for sensing the positions of compact medical device, such as catheters having narrow dimensions.

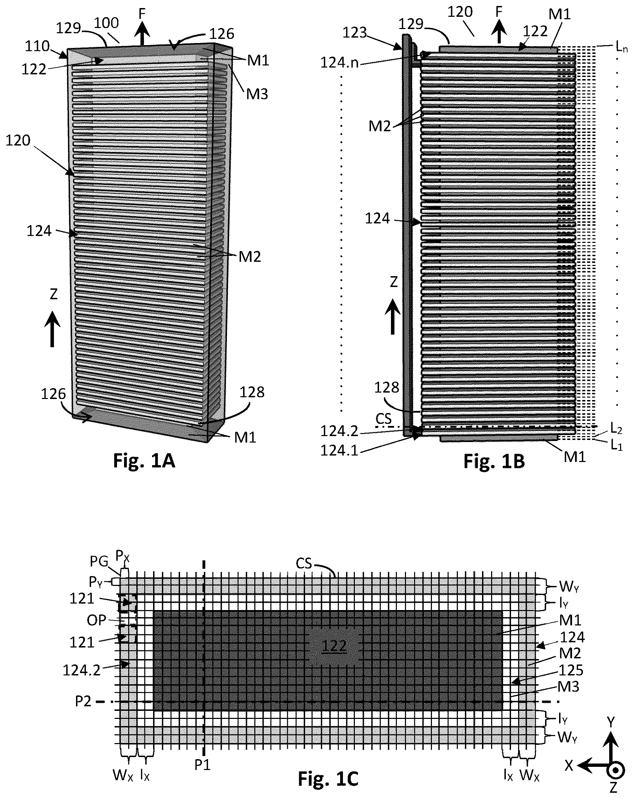

With reference made together to **Figs. 1A** and **1B** an example of an electric component **100** including a coil **120,** according to an embodiment of the invention, is illustrated. The component **100** includes a body **110** with a at least one coil **120** defined/fabricated therein by multi-material 3D printing.

According to the technique of the invention the electric component **100** (and the coil(s) **120** therein) is fabricated by 3D printing of s of at least three different 3D-printable materials **M1** to **M3** including:
- At least one type of magnetic material **M1** which is used/3D-printed to form at least a magnetic channel(s) **129** of the coil **120,** which typically includes magnetic core **122** of the coil **120.** Optionally, the magnetic channel **129** also includes magnetic flux collectors **126** which may be coupled to the magnetic core **122** from opposite ends thereof and have larger surface/cross-section area than the core facilitating channeling of higher magnetic flux through the core to thereby improve the coil's sensitivity to magnetic fields from a desired magnetic field direction.
- At least one type of conductive (generally non-magnetic) material **M2** which is used/3D-printed to form a conductive channel **128** of the coils **120** including conductive windings **124** and contact terminals **123** thereof (the contact terminals are not specifically shown in **Fig. 1A****);** and
- At least one type of dielectric/non-electrically-conductive material **M3** which is used/3D-printed to form a bulk of the body **110** of the electric component **100** at regions from which the magnetic and conductive materials are excluded.
Spacings between adjacent windings/turns **124** are occupied by 3D printed non-electrically-conductive material. The non-electrically-conductive material may for instance be the non-magnetic dielectric material **M3** which is 3D printed in the bulk of the body **110** of the electric component **100** and/or the magnetic material **M1** used for printing the magnetic channels in cases where it is non-electrically conductive (i.e. sufficiently electrically insulative material).

**Fig. 1A** illustrates a perspective view of the electric component **100** with the coil **120** therein, and with the bulk material **M3** of the body **110** shown/exemplified in translucent color/shade so that the coil **120** therein is visible. **Fig. 1B** illustrates a side view of a part of the coil **120** which is 3D printed within the electric component **100** shown in **Fig. 1A****,** while excluding (not showing) the bulk material **M3** of the body **110** of the electric component **100** and also excluding (not showing) the optional flux collectors **126** in order to illustrate the coil **120** with better clarity.

As illustrated the coil **120** includes the magnetic channel **129,** which includes in this example the core **122** of the coil **120** with two optional magnetic flux collectors **126** arranged from opposite ends thereof. The coil **120** also includes an electric channel **128** arranged/configured therein to yield induced voltage through the electric terminals **123** (not specifically shown in **Fig. 1A****)** of the electric channel **128** in response to magnetic flux propagation through the core **122.**

To this end, it should be understood that in various embodiments the magnetic channel **129** and the electric channel **128** may have different shapes. Generally, at least one of the magnetic **129** and electric **128** channels has coiled/twisted geometry (e.g. a helical or otherwise twisted geometry) and is wrapped/surrounded or otherwise twisted about the other one of the magnetic **129** and electric **128** channels, in order to yield the induced voltage in the electric channel **128** in response to magnetic flux propagation through the core **122** of the magnetic channel **129.**

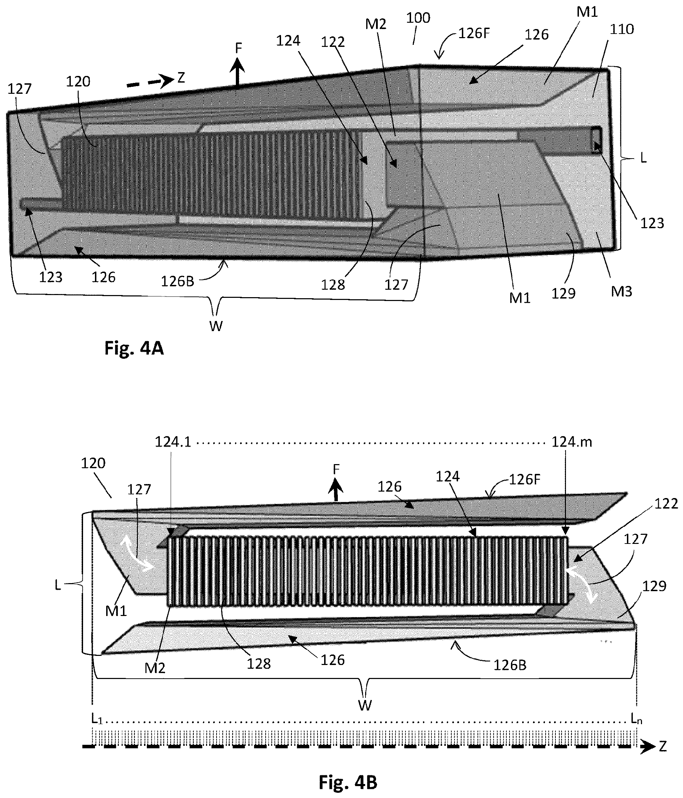

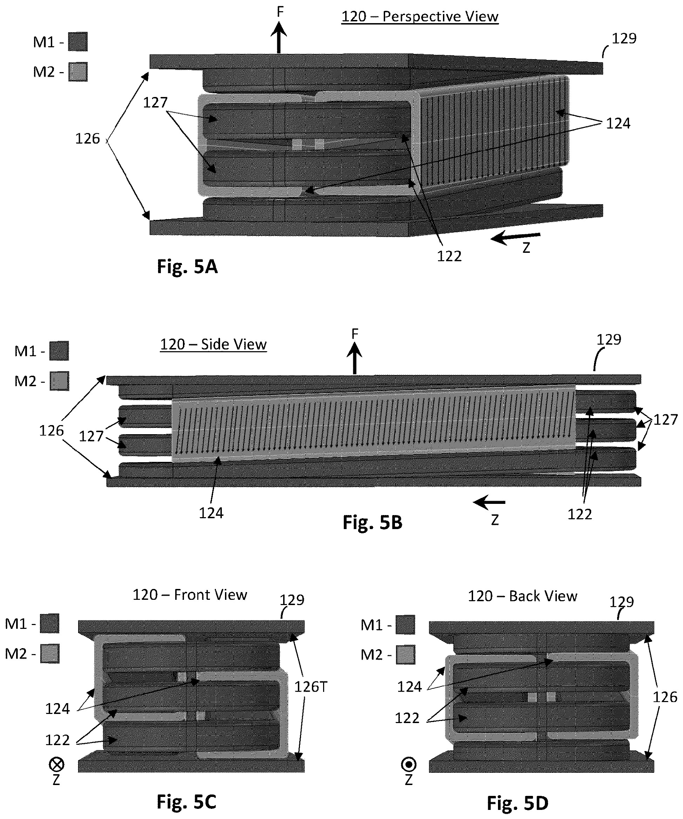

In this regard, in the non-limiting example of **Figs. 1A and 1B****,** a magnetic core **122** having a straight/rod-like shape is exemplified in the magnetic channel **129,** while the electric channel **128** has conductive windings **124** turned/twisted/coiled such that it form a plurality of turns **124.1 to 124.n** wrapped/surrounded about the magnetic core **122** of the magnetic channel **129,** so that induced voltage is produced in the electric channel **128** in response to magnetic flux through the core **122** of the magnetic channel **129.** In this connection it should be noted that the magnetic channel **129** and/or the magnetic core **122** thereof are not necessarily shaped to form straight/rod-like core and may be 3D printed with other shapes that are turned/twisted/coiled within the body of the electric component **100.** For instance, the core **122** may be formed with helical, meander-like or coiled shape with the windings **124** surrounding multiple regions thereof. Such core shapes may be used in order to facilitate generation of sufficiently high induced voltage of the coil **120** desired sensitivity of the coil **120** to magnetic fields from a desired magnetic field direction/axis **F,** while with desired dimensional constraints of the coil **120** or of the electric component **100** in its entirety. For instance, in **Figs. 4A** and **4B** described in more details below, an example of a 3D printed coil **120** having low/small aspect ratio between its length along its magnetic axis **F** and its width/lateral dimension (which is in this case the direction **Z** of the 3D printing) and; in this example the magnetic channel **129** of the coil **120** has a twisted shape and turns **127** of the channel **129** between the flux collectors **126** and the core **122** such that the core **122** extends along the longer, lateral, dimension of the coil **120** and has a greater length to accommodate conductive windings **124** surrounding it, than would have being in case of a straight core extending along the shorter length of the coil **120** along magnetic field axis **F.** In another examples, shown for instance in **Figs. 5A** to **8B** described in more details below, various 3D printed coils **120** according to embodiments of the invention are illustrated with their magnetic channels **129** (e.g. magnetic cores **122)** having twisted shapes of either a helical, multi/double helical, meander, and/or coiled, shapes; whereby these twisted core shapes facilitate a greater length of the core **122** passing through windings of the electric channel 128 or otherwise surrounding the electric channel **128** thereby increasing the amount of magnetic flux inducing voltage in the electric channel **128** to improve the sensitivity of the coils while facilitating various desired dimensional constraints thereof (to enable fitting them within small medical devices).

Optionally, as also exemplified in **Fig. 1A****,** the magnetic channel **129** of the coil **120** includes flux collector(s) **126** magnetically coupled-to the core **122** of the channel **129.** The flux collector(s) **126,** typically a pair of them coupled to opposite sides of the core **122,** are configured with respective relatively large facets/surfaces perpendicular to the magnetic axis **F** of the coil **120** (e.g. typically larger than the cross-section area of the core **122)** such that they can collect magnetic flux propagating along the direction of the coil's magnetic axis **F,** and channel the flux through the core (thereby improving the sensitivity of the coil). As indicated the flux collectors are optional, and are included in the magnetic channel **129** in some embodiments where the cross-section of the magnetic core **122** is small/narrow and/or when the magnetic core **122** does not extend in direction substantially parallel to the magnetic axis **F.**

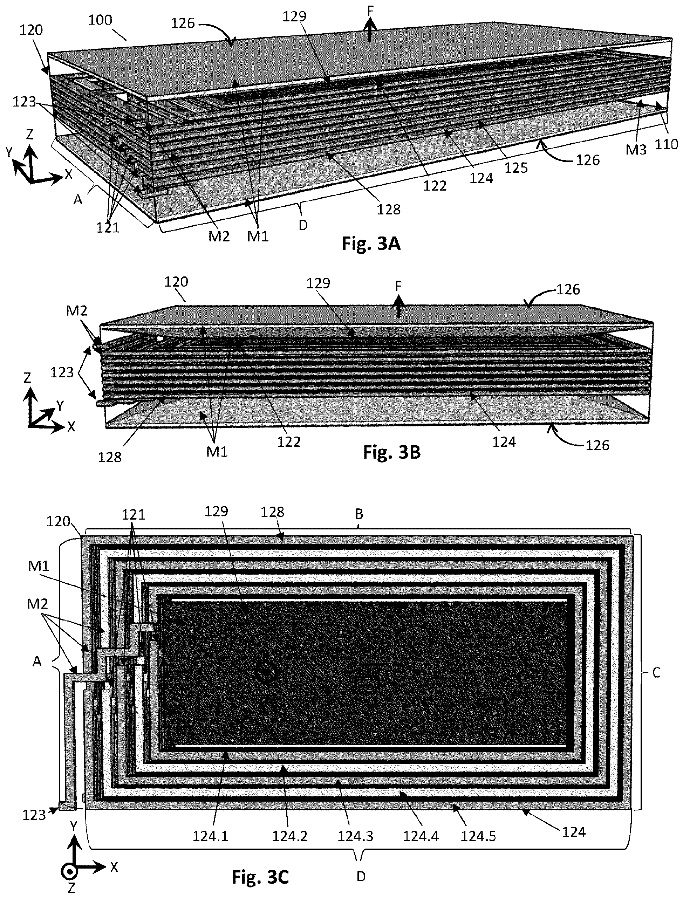

Optionally, as also exemplified in **Fig. 1A****,** the electric channel **128** of the coil **120** includes conductive windings **124,** including a plurality of turns surrounding the magnetic core **122,** in order for voltage to be induced therein in response to magnetic flux through the core **120.** The conductive windings **124,** are optional, as in some implementations, as indicated above, the magnetic core **120** may be itself winded/turned to surround the electric channel **128** such that magnetic flux propagating therein circumferences the electric channel **128** and induces voltage therein. In various embodiments, the conductive windings **124** may be configured/3D-printed to surround the magnetic core **120** in a coiled shape/path (as illustrated in the example of **Fig. 1A****)** and/or with spiral shape/path within one or more of the printed layers, and/or with the shape of multiple concentric coils (as illustrated in the example of **Figs. 3A to 3I****).**

Generally, as would be appreciated by those versed in the art, the electric component **100** of the present invention may include/be-fabricated-with one or with a plurality of coils **120** therein, as well as optionally with other electric circuit elements (not specifically illustrated) such as capacitors and/or resistors and/or electrical connections between them. A person of ordinary skill in the art will readily appreciate how to fabricate/3D-print such other electric circuit elements within the electric component 100 of the invention, while optionally utilizing additional 3D printable materials not specifically exemplified herein, such as electrically resistive material for fabrication of resistors, and or material of selected dielectric properties to be 3D printed in between capacitor's conductive plates.

According to some aspects of the present invention, the electric component **100** of the invention is fabricated utilizing 3D printing method (additive manufacturing technology) that includes a layer-by-layer deposition (and optionally curing), along a printing direction **Z,** of the at least three printable materials **M1** to **M3** indicated above. Typically the at least 3 types of printable materials include: the magnetic material(s) **M1**, which is used for printing the magnetic channels **129** of the coils **120** (including the magnetic cores **122** and the optional flux collectors **126** thereof); the conductive material(s) **M2** which is used for printing the electric channels **128** of the coils (including the conductive windings **124** and the electric contacts/terminals thereof); and non-magnetic electrically insulating (dielectric) material, which is 3D printed to form the bulk of the body **110** of the electric component **100,** as well as typically/optionally to fill the spacings between the windings **124** of the coils to electrically insulated between them.

The terms *magnetic- material* and/or *particles* is used herein to designate materials of high relative magnetic permeability µᵣ, for example having relative permeability preferably of µᵣ ≥100 relative to the vacuum permeability µ₀, and more preferably in some cases in the order of µᵣ ≥500, or even in the order of µᵣ ≥5000. Conversely the terms *non-magnetic- material* and or particles are used herein to designate materials of low relative permeability µᵣ which is substantially lower than that of the magnetic materials used in the electronic components' fabrication and typically in the order of µᵣ ~ 1.

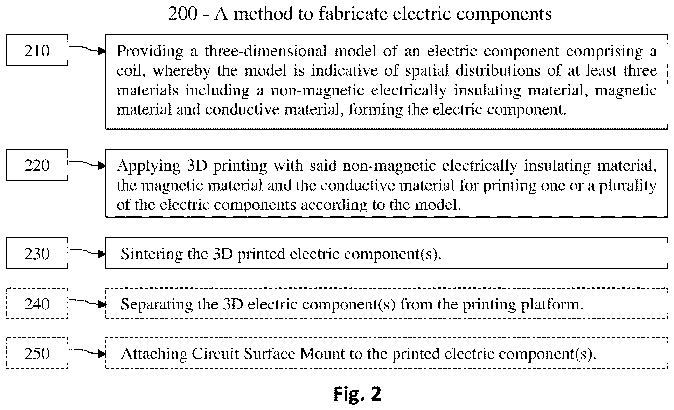

With reference to **Fig. 2****,** a flow diagram of a method **200** to fabricate the electric component **100** according to some embodiments of the present invention by multi-material 3D-printing is illustrated. The multi-material 3D-printing in this case utilizes at least three different materials **M1 to M3** for printing the electric component(s) **100.**

In operation **210** of method **200,** a model of one or more electric component **100** according to embodiments of the present invention are provided. The model may include one or more electric component **100** configured with the principles of the invention as described above with reference to **Figs. 1A** and **1B** and/or as described below with reference to **Figs. 3A** to **8B****.** The model is indicative 3D spatial distribution (voxel maps) of the at least three materials, **M1 to M3,** which are to be 3D-printed to fabricate the electric component(s) **100** of the present invention. Particularly the model is indicative of the respective spatial distributions of the three materials **M1 to M3** that should be 3D printed to form an electric component **100** with at least one coil **120** according to the invention.

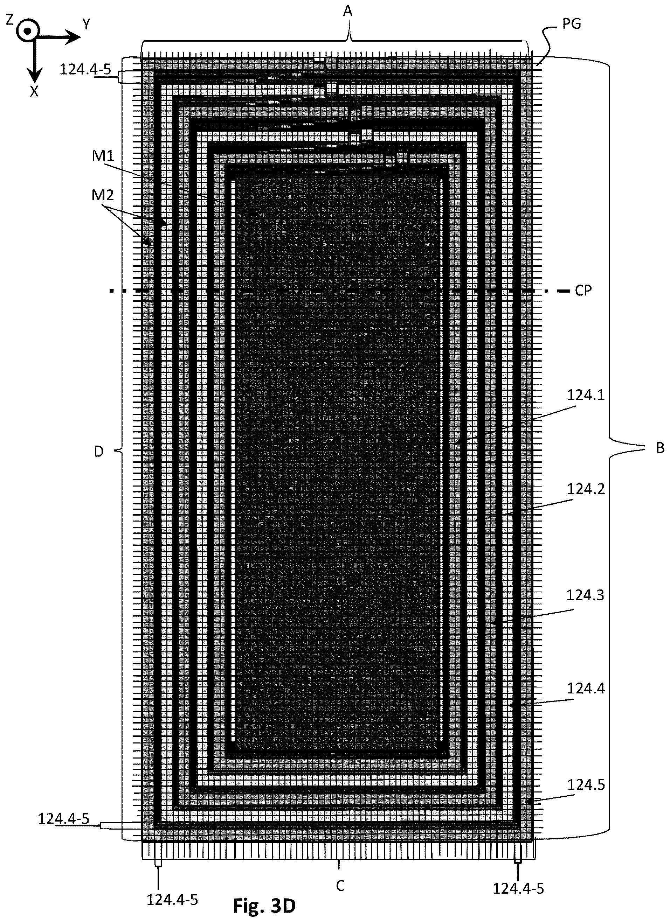

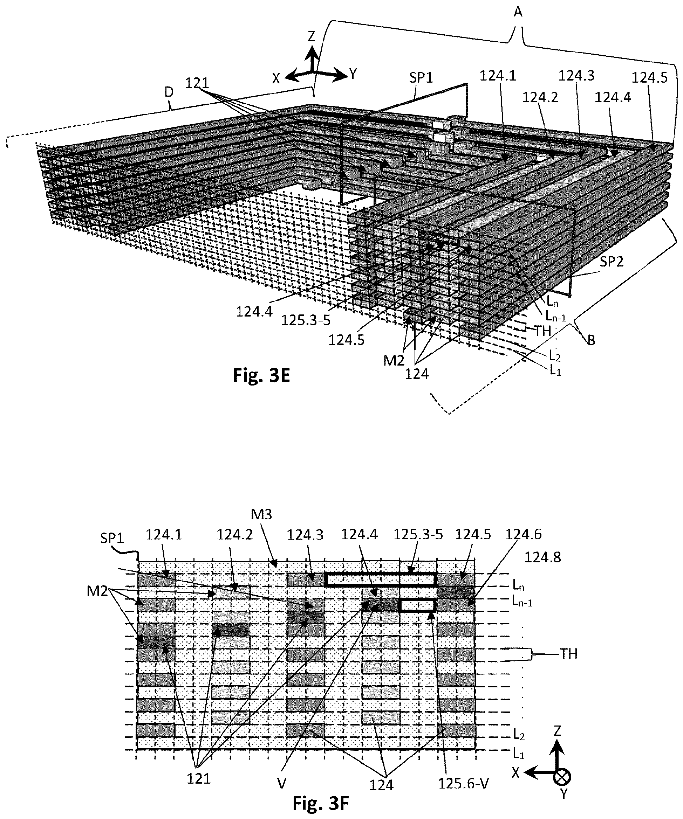

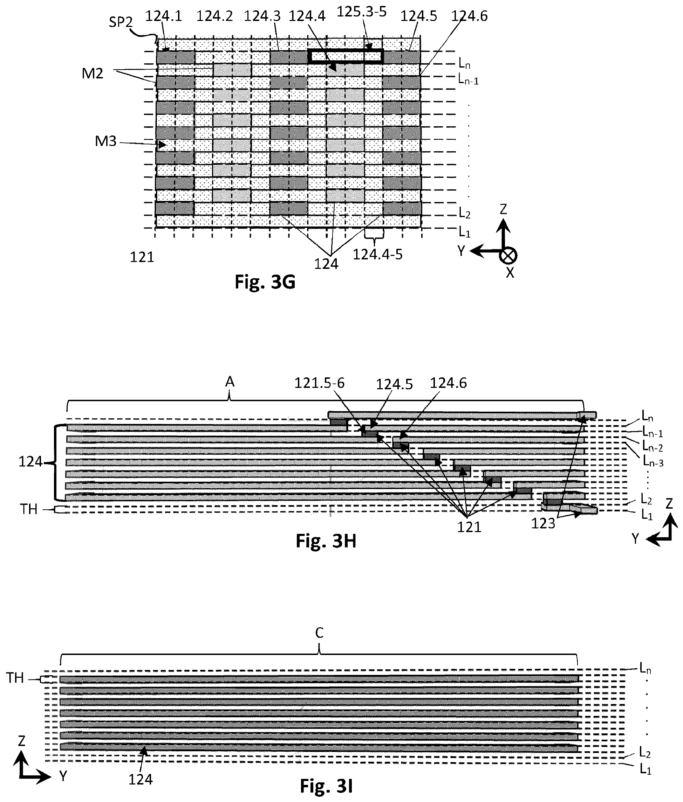

Typically, the multi-material 3D-printing according to method **200** operates by successive printing of layers, one on top the other, along the printing direction **Z** (see for instance the layers indicated **L₁, L₂ to Lₙ** in **Figs. 1B****,** **3D****,** **3E****,** **3H** and **3I****).** The spatial distributions of the three materials **M1 to M3** in the model is such that the printing results with one or more electric components **100** each including at least one coil **120** configured according to an embodiment of the present invention.

In operation **220** of the method **200,** a multi-material 3D-printing is applied to print the one or more magnetic field sensors **100** based on the spatial distributions of the three or more materials **M1** to **M3** in the model. The multi-material 3D-printing may be carried out for example by utilizing a photopolymerization 3D-printing technique to print layers **L₁, L₂ to Lₙ** that are composed of a combination of several materials having different electromagnetic properties (e.g., two three or optionally more than three materials with different electromagnetic properties may be printed in each layer). In a typical example, 3D printing of the electric component **100** described above involves printing of spatial distributions of the at least three materials **M1** to **M3** having the different electromagnetic properties.

To this end in various embodiments the electrically conductive material **M2** may include for example Silver and/or Copper and/or Palladium, and/or Silver-Palladium alloy. The magnetic material **M1** may include for example Ceramic-Ferrite material and/or Metal Material. In some embodiments the magnetic material **M1** is a soft magnetic material having relative permeability µᵣ of at least 100 or above. Preferably the magnetic material **M1,** particularly that used for printing magnetic channel **129** having coiled/twisted or otherwise non-straight shapes, is a material having relative permeability µᵣ in the order of 500 or above, and even more preferably in the order of 5000 or above, in order to facilitate efficient channeling of magnetic flux through the folds/curves **127** thereof. The non-magnetic dielectric (electrically insulating) material **M3** (also referred to herein as the bulk material) may for instance include ceramic and/or glass-ceramic.

The 3D printing of the electric component(s) **100** may be implemented for example based on the principles of Vat Photopolymerization, utilizing for instance stereolithography (SLA) or digital light processing (DLP) for the pattern implementation/projections. The invention is not limited to these specific technologies and may generally be implemented by other multi-material 3D printing technique as may be developed to be suitable for the fabrication of the electric components according to the invention.

With the technique of the invention the electric component(s) **100** with small feature sizes can be printed utilizing 3D printed layers with layer-thicknesses in the scale down to microns (e.g., sintered layer thickness may be about 8-10 microns). The pixelated in-layer resolution of the printing may be with pitch/widths of the printed voxels in the order of about 12-25 microns laterally within the layers (i.e. with respect to the lateral coordinates **X, Y).**

In some embodiments of the invention functional features of the electric component(s) **100** are printed with minimal in-layer feature size ( of at least two voxels, in order to avoid malfunctions of the electric component(s) **100** due to misprints of single/individual voxels. Accordingly, features of the electric component(s) **100** which are printed within a layer may be printed with characteristic in-layer lateral sizes as small as about 24-50 microns or less (this is considering that the lateral pitch/widths of the voxels within the layer is of about 12-25 microns and considering the preference to print each important/functional feature with minimal in-layer feature size of at least two the voxels).

In this regard, reference is made to **Fig. 1C** showing a cross section of the electric component(s) **100** shown in **Fig. 1B** along the plane/printed-layer **CS** marked there, which passes across the winding turn **124.2.** As shown the turn **124.2** is not closed (opened at region **OP)** such that it is connected, via conductive vias **121** to other turns of the conductive windings **124** above and below it (in this manner forming the conductive windings **124** in the coil's electric channel **128).** It should be noted that the term via (e.g. referring to elements marked **121** in the figures) is used herein to designate parts/portions of the conductive channel **128** (e.g. integral parts thereof) which are 3D printed to electrically connect between turns of the conductive windings **124** that are printed in above or below one another in different layers.

**Fig. 1C** shows a pixels' grid **PG** illustrating the voxel distribution within a certain printed layer of the electric component **100,** with **P_{X}** and **P_{Y}** marking the voxels' lateral width/pitch along each of the lateral (in-layer) **X** and **Y** dimensions (which, as said, may be in the order of 12-25 microns when printing compact electric components). As illustrated, in this non-limiting example, the electric channel **128** including the turns of the windings **124** of the coil **120** (which are a functional element thereof) are printed with minimal in-layer feature size of at least two voxels. Also, insulation **125** separating parts of the electric channel **128** from other parts thereof (e.g. such as between turns **124.1 to 124.n)** of the windings **124,** or from other optionally conductive elements of the electric component (such as the core **122** which may be conductive in this non-limiting example), are printed with minimal in-layer feature size of at least two voxels.

In this connection the phrase *minimal in-layer feature size* is used herein to design the minimal lateral size in voxels/pixels, of a functional feature of the electric component along any lateral (X and/or Y) directions of the 3D-printing within each layer in which it is being printed. This means that in this example, by "cutting" any part of a functional feature, such as the electric channel **128** and/or the insulation **125,** across any printed layer with orientation of the cut plane perpendicular to the printing direction (i.e. parallel to the layer), the visible dimensions of the cross-section area of the functional feature within the cut (within the layer) would be not less than two 3D-printed voxels/voxels, which are filled (or supposed to be filled in case of a misprint) with the suitable material (e.g. **M2** for the conductive channel **128** and **M3** for the insulation **125)** along each of the X and Y lateral dimensions/axes. In other words, the minimal in-layer cross-section area of any functional feature is at least 2X2 voxels/pixels printed within the layer. In this regard it is noted that in at least some of the 3D printing techniques, features of size of a single pixel/voxel within the printed layer (e.g. single pixels/voxels isolated from any sequence of consecutive pixel/voxels of the same material within the printed layer), may occasionally be misprinted. Accordingly, by ensuring that the functional features of the electric component **100** of the invention are printed with the *minimal in-layer features size* of at least two voxels along each lateral direction X and Y, provides for mitigating such misprints of single voxels, and ensures that even if a single voxel is misprinted within the layer, the respective functional feature in which the misprint occurred will still operate properly. With respect to the insulation **125,** it should be noted that in various embodiments, where the insulation **125** is 3D printed with the same material as the bulk of the monolithic body **110,** there may not be any specific features to distinguish the insulation **125** from other parts of the monolithic body **110.** Therefore it should be clarified that the term insulation **125,** is used herein considering its functional property, to electrically insulate between to conductive features of the electrical component **100,** such as between winding turns, and while considering this functional property, any set of voxels of electrically insulative material which are printed within each layer along the shortest path(s) between to conductive features in the layer, should be considered as a part of the insulation **125** between the said conductive features. Accordingly, with this definition, the *minimal in layer feature size* of the insulation is such that the shortest insulative path(s), in voxels, between conductive features/voxels within a layer extend through at least two voxels of the layer.

Accordingly, any individual misprint of single voxel in the electric channel **128** will not cause electrical disconnection in the electric channel **128,** and any individual misprint of single voxel in the insulation **125** will not affect electric shortcut in the electric channel **128.** In like manner it should be understood that also the magnetic channel **129** is printed with the minimal in-layer features size of the at least two voxels (in some implementations it is 3D printed with in-layer features sizes substantially larger than two voxels in order to facilitate efficient propagation of magnetic flux therethrough).

To this end, in this example the turns of the electric channel **128** is printed with in-layer lateral widths **W_{X}** and/or **W_{Y}** of at least two voxels along at least one of the lateral dimensions **X** and **Y,** and with height of one layer so that they have at least said minimal *in-layer feature size* of the two voxels. Accordingly, any individual voxel misprint in the winding will not cause electrical disconnection in the electric channel **128** and will not disrupt the operation of the coil **120.** Additionally, as in this specific non-limiting example magnetic material **M1** of which the core **122** is made is electrically conductive metal (i.e. note that as described below the core **122** and/or the magnetic channel **129** are not necessarily made of conductive metal - see the discussion below regarding the printing of the magnetic channel **129** with electrically insulating ceramic ferrite material), and therefore insulation **125** is printed between the conductive windings **124** and the core **122** to prevent shortcut of the windings **124.** The insulation **125** is also printed with respective in-layer lateral widths **I_{X}** and **I_{Y}** of at least two pixels along the lateral dimensions **X** and **Y,** such that its minimal *in-layer feature size* is at least two voxels. Accordingly, any single voxel misprint in the insulation **125** will not cause shortcut in the electric channel **128** and will not disrupt the operation of the coil **120.** To this end, the method **200** facilitates robust and reliable fabrication of the electric components, by utilizing printing each functional feature of the electric component **100** with *in-layer feature size* of at least two voxels.

For carrying out the 3D printing, curable resins corresponding respectively to the at least three materials **M1, M2** and **M3** are provided. More specifically the curable resins used in the 3D printing of the magnetic field sensor **100** of the invention include for example the following:
- A magnetic material resin which includes particles of a soft magnetic material magnetic material **M1** suspended in curable polymer binder. The particles of the magnetic material **M1** may include for example particles of Ceramic-Ferrite material. It should be noted that alternatively in some embodiments the particles of the magnetic material **M1** may include particles of magnetic Metal Materials such as Iron alloy, iron-silicon alloy, iron-aluminum-silicon alloys, low-carbon steels, nickel-iron alloys, iron-cobalt alloys, ferrites, amorphous alloys, as well as in some cases Nickel-Cobalt Alloy (particularly in cases it has soft magnetic qualities). In embodiments where miniature coils are fabricated within the monolithic body **110** the particles of magnetic material used may be provided for example with characteristic sizes in the micron to submicron scale, particularly in cases where the magnetic channels **129** of the coils manifest delicate features.
- A conductive material resin which includes particles of conductive material **M2** suspended in curable polymer binder. The particles of the conductive material **M2** may for instance include Silver material, Copper material, Palladium material, and/or Silver-Palladium alloy. As specific choice of suitable conductive material may generally depend on the required temperatures of the sintering/firing process required to finalize the printing of the electric components and may thus depend on the types of magnetic and dielectric materials. In embodiments where miniature coils are fabricated within the monolithic body **110,** the particles of conductive material **M2** used in the printing may have characteristic sizes in the submicron scale and as small as in the nanometer scale.
- A dielectric and non-magnetic material resin which includes particles of non-magnetic dielectric material **M3** suspended in curable polymer binder. The particles of the non-magnetic dielectric material typically include ceramic or glass-ceramic particles which can withstand the high sintering/firing temperatures required for sintering the printed magnetic and the conductive materials, **M1** and **M2.** Also, here the size of the particles may be selected according to the minimal feature sizes that should be occupied by the dielectric material/bulk of the magnetic field sensor, and for fabrication of miniature coils may be selected for instance in the order of nanometer to submicron scale.

In regards to the material selection in the above, it should be noted that although printing magnetic channel **129** with magnetic metal material (e.g. electrically conductive) is generally possible, in various embodiments printing the magnetic channel with an electrically insulating ceramic ferrite may be preferred. One reason for that is that the use ceramic ferrite facilitates achieving more compact arrangement of magnetic and electric channels, **129** and **128.** This is because soft magnetic metal materials (non-ceramic ferrite) that are suitable for use for the magnetic channel printing, are typically conductive. Therefore, a magnetic channel **129** printed with a non-ceramic ferrite, should be spaced from the electric channel **128,** at least at some places, in order to prevent short circuit, which might impact the achievable miniaturization of the magnetic field sensor **100** and/or require more complex design thereof. Therefore, in some embodiments preferably ceramic ferrite material is used for the printing of the magnetic channels **129.**

Thus, utilizing the at least three curable resins described above, 3D printing of the magnetic field sensor **100** may be performed layer by layer along a printing direction **Z** utilizing for example photopolymerization multilateral 3D printing techniques such as SLA or DLP. The printing direction **Z** is typically selected such that it is substantially perpendicular to planes spanned by the turns of the conductive windings **124** of the coils **120.** To this end, as indicated above, the choice of the printing direction **Z** may have particular importance in the printing of miniature magnetic field sensors **100** because the lateral feature resolution of the printing in the X-Y plane is lower than the "vertical" resolution of the layers obtained along the printing direction **Z.**

To this end, typically the 3D printing of each layer of the sensor **100** typically includes the following (not necessarily in that order):
- Deposition/printing and curing the magnetic material **M1** at regions of the layer corresponding to the magnetic channels **129** of the coils **120;**
- Deposition/printing and curing the conductive material **M2** at regions of the layer corresponding to electric channel **128** of the coils **120;** and
- Deposition/printing and curing the non-electrically-conductive (dielectric) non-magnetic material **M3** to form the bulk of the monolithic body **110** of the sensor **100.**

It should be noted that in some embodiments, the 3D printing is conducted such that the conductive material **M3** of the coils **123** is fully embedded within the bulk of electric component's body **110** in order to protect the conductive material **M3** against degradation (e.g. oxidation) due to environmental conditions, particularly during the high temperature sintering **230** which follows layer by layer material deposition and curing.

Thus, by the consecutive printing and curing of successive layers in operation **220.** according to the spatial distribution of the materials in the sensor's model, one or more 3D structures of one or more sensors **100** each including at least one longitudinally oriented coil and at least one transversely oriented coil electric coil **220** are obtained.

Method **200** further includes operation **230** for sintering the one or more 3D structures of the one or more electric components **100** printed in operation **120.** The sintering is generally performed at temperature sufficient for burning or driving off polymers (binding polymers) from the one or more magnetic field sensors **100** as well as firing ceramic or glass ceramic materials such as **M3** and optionally **M1** which are used in the printing. To this end, after the sintering the material density of the printed ceramic and metal materials (e.g., **M1, M2** and **M3)** of the electric components **100** approach 100%. For example, in embodiments where the conductive material **M2** mainly includes Silver, the sintering may be performed at temperatures below the Silver melting point (e.g., at temperatures not exceeding 961°C; preferable in this case the sintering temperature may be limited up to about 900C in order to achieve a stable sintering process). In other embodiments where the conductive material includes a Silver-Palladium alloy, the sintering may be performed at temperatures below the alloy melting point (the specific melting point depends on the ratio of silver and palladium in the alloy and depending on the composition the melting point is generally between the melting point of silver, 961°C, and that of palladium, 1,555°C). In another example, in embodiments where the conductive material **M2** includes Copper, the sintering may be performed in an Oxygen deprived environment (so as to avoid oxidation of the Copper), and at temperatures below the 1084°C Copper melting point.

In this connection, it should be noted that in some embodiments of the present invention the method **200** is carried out for 3D-printing electric components **100** in which miniature coils having features of micron scale size are embedded. For instance, the 3D printed layers may be printed with thicknesses in a range of about 5 to 15 µm (e.g., 9µm), depending on the specific model design and 3D-Printing technology used. Accordingly, a pitch of the turns of the windings **124,** which may be in the order of twice the pitch of the 3D printed layers, may be within the range of about 10µm to 30µm along the printing direction **Z** of the 3D printed layers (e.g., typically turns' pitch of about 18 µm).

Further, optionally (depending on the specific printing technology used), the method **200** may include operation **240** for separating printed electric components **100** from the building/3D-printing platform (e.g., **201** shown in **Fig. 1E).** Generally, depending on the type of building platform (e.g., and whether it can withstand the sintering **230),** the operation **240** may be performed before or after the sintering **230.** Indeed, in some embodiments of the invention, particularly where miniature/delicate electric components are printed, which may be damaged by carrying out their separation from the building platform before the sintering, a suitable building platform may be used which can withstand the temperatures of the sintering **230,** and the electric components' separation from the platform may be conducted post sintering so as to avoid/reduce damage to the sensors during the separation process.

In view of the above, method **200** of the present invention provides a novel and inventive technique for fabrication of electric components **100** each including one or more coils **120** as described above. The method **200** is suited for fabrication of miniature electric components **100** (with features sizes as small as few microns) that are operable as/in a magnetic-field-based positioning sensors and suited for use in compact/narrow medical devices such as catheters. The method **200** may be used in mass production to simultaneously 3D-print large pluralities of miniature such electric components **100** built on the same platform (e.g., typically between hundreds and several thousand electric components **100** may be printed using for instance a vat photopolymerization technology). Each electric component **100** may have for example a ceramic/glass-ceramic body **110** embedding therein one or more highly sensitive open magnetic circuit coils **120.** The coils **120** may be fabricated with small dimensions, for example in the order of ~0.5 mm to ~2 mm and may provide high sensitivity/induced-voltage in response to magnetic fields, for example in the range ~0.1 to 1 µV/(Gauss*Hz).

Reference is now made to **Figs. 3A** to **3I** illustrating a 3D printed electric component **100** including at least one coil **120** configured according to an embodiment of the present invention. The electronic component **100** includes a magnetic channel **129** and a conductive channel **128** of the coil **120.** The magnetic channel **129** and conductive **128** channels are 3D printed with magnetic material **M1** and electrically conductive material **M2** respectively. The magnetic channel **129** includes a magnetic core **122** of the coil **120,** and the conductive channel **128** includes an arrangement of conductive windings **124** with a plurality of turns surrounding the magnetic core **122** (e.g. some of the turns are individually marked with reference numerals **124.1** to **124.6** in the figures). The body/bulk **110** of the electric component **100** is 3D printed with non-magnetic electrically insulating material **M3.** To this end the electrical insulation **125** between the turns of the windings **124** is in this example 3D printed with the same non-magnetic electrically insulating material **M3** used for printing the bulk of the body**110.**

In the figures **3A** to **3I**: **Fig. 3A** shows a perspective view of the complete electric component **100;** **Fig. 3B** is a side view of the coil **120** within the electric component **100** in which the non-magnetic electrically insulating material **M3** is not illustrated for clarity of the figure; **Fig. 3C** shows a top view of the coil **120** within the electric component **100,** with the flux collectors **126** omitted/not-shown for clarity; **Fig. 3D** shows a top view of the coil **120** without showing the electric terminals **123** and showing a pixel/voxel grid **PG** illustrating the distribution voxel of the 3D printing as captured from the top side of the coil **120;** **Fig. 3E** illustrates a cross-section of the windings **124** of the coil **120** with the insulation **125** between them across lateral sides **B** and **D** of the coil **120** (in which no conductive vias **121** between turns are accommodated), taken along the cross-section plane **CP** shown in **Fig. 3D****;** **Figs. 3F** and **3G** illustrate section cuts taken along the respective section planes **SP1** and **SP2** of the coil **120** shown in **Fig. 3E****,** showing the windings **124** with the insulation **125** between them respectively across the lateral side **A** (in which the conductive vias **121** between turns in different layers are accommodated) and across the lateral side **C** of the coil **120** (in which now conductive vias **121** are accommodated); and **Figs. 3H** and **3I** are two respective side views showing some of the turns of the conductive windings **124** (without showing the insulation **125** between them), which are taken respectively from lateral side **A** of the coil **120** along which conductive vias are accommodated between the windings **124** and from lateral side **C** of the coil **120** along which conductive vias are not accommodated in this example. In these figures, where applicable, axes indicating the longitudinal direction **Z** (being the layer-by-layer-printing direction of the electric component **100)** and the lateral directions **X** and **Y** perpendicular thereto, are illustrated. Also, where applicable, guide lines illustrating the 3D printed layers **L₁** to **Lₙ** of the electric component **100,** the lateral side **A, B, C** and **D** thereof, and the magnetic axis **F** of the coil **120** therein, are illustrated in the figures.

For example, as illustrated in **Fig.3H****,** the via **121.5-6** is printed in layer **Lₙ₋₂** to connect between the turns **124.5** and **124.6** which are 3D printed in the layers above and below it, **Lₙ₋₁** and **Lₙ₋₃**, respectively.

As indicated above, preferably according to some embodiments of the present invention, functional features of the 3D printed electric component(s) **100** are **3D** printed with minimal *in-layer feature size* of at least two voxels.

In the embodiment of the electric component **100** of **Figs. 3A** to **3I**, a configuration of the coil's **120** windings **124,** which facilitates compact arrangement of large number of windings' turns, while maintaining a minimal *in-layer feature size* of at least two voxels in the windings' turns **124** and in the electric insulation **125** between them, is illustrated. This configuration thus facilitates the reliable 3D printing of highly sensitive and compact coil(s) in the electric component, since on the one hand any individual voxel misprint in the electric channel **128** or the insulation will not disrupt proper operation of the coil, while on the other hand it provides for stacking large number of windings' turns **124** surrounding the magnetic core **122** thereby facilitating the high sensitivity of the coil **120.**

To achieve that one or more of the following arrangement features are implemented in the arrangement of the 3D printed turns **124.1** - **124.n,** and the insulation **125** in the spacings between turns:
**a.** The conductive channel **128** (including the turns of the conductive windings **124)** as well as the electrical insulation **125** between the windings' **124** turns, are each 3D printed with the minimal in-layer feature size of the at least two voxels; This contributes to the reliability of the electric component **100,** and or of its production by the 3D printing as discussed above.
**b.** The turns, e.g. **124.1** - **124.n** or some of them, of the conductive windings **124** are arranged/3D-printed planarly within the printed layers **L₁** to **Lₙ**. Accordingly since the thicknesses **TH** of the printed layers/voxels (along the printing direction **Z)** is typically smaller than the lateral X, Y dimensions of the voxels, printing the turns plenary within the layers facilitates stacking greater number of turns.
**c**. A plurality of concentric turns are printed at least some of the layers **L₁** to **Lₙ** so as to facilitate printing of greater number of turns surrounding the core **122.** For instance, in this example turns **124.1, 124.3** and **124.5** are concentric turns printed plenary within layer **Lₙ**; and turns **124.2** and **124.4** are concentric turns printed plenary within layer **Lₙ₋₁**.
**d.** A plurality of concentric turns are printed in consecutive layers so as to facilitate printing/stacking of a greater number of turns surrounding the core **122.** For instance, in this example turns **124.4** and **124.5** are concentric turns printed plenary within the consecutive layers **Lₙ₋₁** and **Lₙ** respectively.
**e.** Concentric turns that lay (3D printed) in consecutive layers and that are concentrically adjacent (in embodiments that such turns exist) are laterally separated, from one or more lateral sides of the concentric, by only one lateral voxel between them. This is in order to facilitate printing/stacking of a greater number of turns surrounding the core **122.** For instance, in the present example the turns **124.4** and **124.5** are concentrically adjacent turns laying respectively within the consecutive layers **Lₙ₋₁** and **Lₙ**. The lateral separation **124.4-5** between them (with respect to the X and/or Y axes) is of only one voxel along the lateral sides **B, C,** and **D** of the electric component 100 (i.e. along the lateral side(s) at which no conductive vias **121** are accommodated), and of two voxels (e.g. at least two) along the lateral side **A** (e.g. along the side where the conductive vias are accommodated), as illustrated in **Figs. 3D** and **3E****.** In this regard, it should be noted that the phrase *concentrically adjacent turns* is used herein to designate turns that are adjacent to one another with respect to the radial direction of the concentric arrangement of turns.. In this regard it should be noted that the invention facilitates minimal lateral spacing of only one voxel between turns (along lateral sides not accommodated with vias **121**) , while maintaining the desired two-voxel minimal in-layer feature size of the insulation **125** between turns. This in turn facilitates compact packing of winding turns high sensitivity of small dimension 3D printed coils. In this regard it is noted that the lateral separation **124.4-5** is printed with single pixel width only from the lateral sides of the concentric (in this example B, C and D) at which there are no vias connecting the concentrically adjacent turns to other/neighboring turns above or below them. Therefore, some embodiments also implement the arrangement feature f., which is described in the following, in order to maximize/optimize the length along the concentrically adjacent turns along which they can be spaced from one another by only one voxel.
**f.** Conductive vias **121** which connect each of the concentrically adjacent turns to other/neighboring turns above or below them, or to other parts of the conductive channel **128,** are preferably arranged from as few lateral sides of the concentric as applicable by the geometry and number of turns of the specific coil (e.g. preferably if applicable from one side only), such that from other one or more sides the concentrically adjacent turns can be laterally spaced from one another by only one voxel while maintaining the minimal in-layer feature size of the insulation **125** of the at least two-voxels between conductive features (e.g. turns and/or vias) in the same layer (as desired for manufacturing reliability of the electric component). In this specific non-limiting example, the vias **121** are all arranged along one lateral side **A** of the concentric turns, whereby in order to maintain the minimal in-layer feature size (at least two-voxels) of the insulation **125** between the turns along that lateral side **A** the lateral spacing between concentrically adjacent turns along that side is of two voxels.

To this end, the combinations of the above features a. to f., and in particular the combination of the features d. e. and f. facilitate compact packing of conductive turns while maintaining the minimal in-layer feature sizes of each functional feature of the coil which is desired for printing reliability. **Figs. 3F** and **3G****,** illustrate respective section cut **SP1** of the windings **124** across the respective lateral sides **A** and B thereof in which the conductive vias **121** are respectively accommodated and not accommodated. As shown in **Fig. 3G** although a lateral separation, such as **124.4-5,** between concentrically adjacent turns in different layers, such as between **124.4-124.5,** is of only one voxel along the sides where no vias are accommodated, the insulation between the adjacent turns within the same layer, such as the insulation **125.3-5** between **124.3** and **124.5** within the same layer, has a lateral width greater than the minimal in-layer feature size, in this example a widths of four voxels along the side(s) at which no vias are accommodated . As shown in **Fig. 3F** since the lateral separation, such as **124.4-5,** between concentrically adjacent turns in different layers, such as between **124.4-124.5,** is of more than one voxel (two in this example) along the sides where vias are accommodated (e.g. side **A** in this case), the insulation between the adjacent turns within the same layer along these sides , such as the insulation **125.3-5** between **124.3** and **124.5** along the side **A,** has also a lateral width greater than the minimal in-layer feature size, in this example a widths of six voxels along the side(s) A at which the vias are accommodated. Otherwise, if for example the lateral spacing **124.4-124.5** would have been made with width of one voxel, as it is along sides B to D, the minimal in-layer feature size of the insulation in the spacing/insulation such as **125.6-V** between the adjacent turn such as **124.4** and **124.6** within the same layer would have been of only one voxel (e.g. due to the presence there of the via between them (e.g. the via indicated **V** in the figure which connects between neighboring turns above one another), and would thereby not satisfy the minimal in-layer feature size condition for the insulation **125.** Therefore in some embodiments of the invention conductive vias **121** which connect between neighboring turns above or below one another are arranged along as few lateral sides as possible whereby the lateral spacing between concentrically adjacent turns along these sides is of at least two voxels, thereby permitting the lateral spacing between the concentrically adjacent turns along other lateral sides to be of only one voxel to yield one the one hand a compact arrangement of the turns and on the other hand electrical insulation with minimal in-layer feature size of at least to voxels facilitating reliability of the coil's fabrication.

In this example, the electric component **100** is 3D printed by successive printing of a plurality of printed layers **L₁, L₂** to **Lₙ** along the printing direction **Z.** Each of the turns, e.g. **124.1** to **124.6,** of the conductive windings **124** is arranged/3D-printed planarly within one of the printed layers **L₁** to **Lₙ**. For example, as shown in **Figs. 3E** to **3I****,** the turns **124.1, 124.3,** and **124.5** are 3D printed planarly within layer **Lₙ** and the turns **124.2, 124.4** are 3D printed planarly within layer **Lₙ₋₁**. To this end in this example the turns are printed in the multitude of the printed layers. The magnetic core is printed such that passes through a consecutive multitude of the printed layers **L₁** to **Lₙ** and surrounded by the turns thereat. The electric component **100,** i.e. the coil **120** thereof, includes electrical insulation **125** that is 3D printed between the turns of the conductive windings **124.** In this non-limiting example the electrical insulation **125** is 3D printed with the same bulk material **M3** which is also used to print the body **110** of the electric component **100.**

As indicated above, in this example the turns (e.g., **124.1** to **124.6)** of the conductive windings **124** and the insulation material **125** between them (as well as the magnetic channel **129)** are 3D printed with minimal in-layer feature size of at least two voxels of the 3D printing. In order to obtain the highly sensitive coil, in this example the windings **124** includes concentrically adjacent turns that are 3D printed in consecutive layers of the 3D printing with lateral separation of one voxel between the concentrically adjacent turns (at least along some lateral sides of the concentric). As indicated above, lateral separation of the one voxel between concentrically adjacent turns is from one or more lateral sides of the adjacent concentric turns (in this example from sides **B to D),** and the conductive vias are 3D printed in at least one other lateral side of the concentric turns (in this example from side A). Along the at least one other lateral side (in this example side **A)** at which the vias **121** are printed/accommodated, the lateral separation between the concentrically adjacent turns is of at least two voxels, in order to maintain a minimal in-layer feature size of the insulation to be at least two voxels.

Reference is now made together to **Figs. 4A** and **4B** illustrating an electric component **100** according to another embodiment of the present invention, which is/includes a coil **120** having a flattened shape with respect to its magnetic axis **F** or in other words a coil **120** having a low/small aspect ratio between its length **L** along its magnetic axis **F** and its width **W** traverse to its magnetic axis **F.** For brevity a coli with such dimensions is referred to herein as *a flattened coil.*

Similarly to the electric component **100** described above with reference to **Figs. 1A** to **1C****,** also in this embodiment of **Figs. 4A** and **4B****,** the electric component **100** includes: a body/bulk **110** that is 3D printed with non-magnetic electrically insulating material **M3;** a magnetic channel **129** that is 3D printed in the body **110** with magnetic material **M1** and includes a magnetic core **122** of the coil **120;** and a conductive channel **128** that is 3D printed in the body **110** with electrically conductive material **M2** and includes conductive windings **124** with a plurality of turns surrounding the core **122.** The and conductive channel **128** is terminated at both ends thereof by electric contacts/terminals **123** (not specifically shown in **Fig. 4B). Fig. 4A** is a perspective view of the electric component **100** and **4B** is a side view of the coil electric component **100** in which the bulk material of the body **110** is not shown.

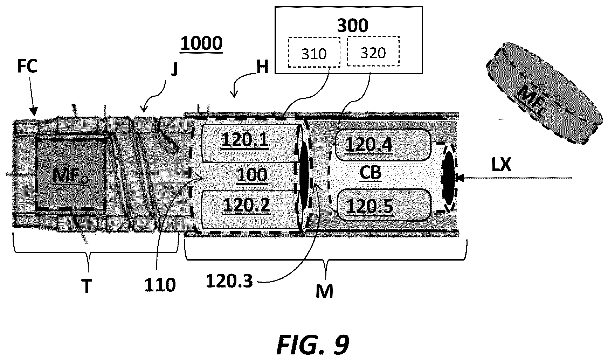

As indicated above, in this embodiment, the required dimensions of the electric component **100** with the coil **120** therein, are such that the electric component **100,** and/or the coil **120** therein, is flattened with respect to the direction of its magnetic axis **F.** In this regard flattened coils, or otherwise coils with such flattened dimensions, are in many cases required for medical devices' position sensing, and in particular are used/fitted in elongated and narrow medical devices, such as catheters, whose positions are often sensed by measuring magnetic field in their surroundings (particularly used for sensing the vector components of the magnetic fields which are perpendicular to the elongated body of the medical device/instrument) - see for instance **Fig. 9** and its related description below.

However, one difficulty of 3D printing of such flattened coils with sufficient sensitivity for applications such as position sensing, is that the number of windings **124** turns than can be 3D printed such that they are stacked along the relatively short length **L** of the coil **120** (i.e. along its dimension parallel to the coli's magnetic axis **F).** Furthermore, as will be appreciated by those versed in the art, due to their geometry, flattened coils with magnetic core have low *apparent permeability* (also known as effective permeability) which may be <2, regardless of high permeability of the magnetic material.

Some aspects of the present invention provide for overcoming this difficulty, and 3D printing flattened coils **120** with sufficient sensitivity. This is achieved by 3D printing the magnetic channel **129** such that it includes at least a pair of flux collectors **126** having respective flux collection facets **126F** and **126B** that are perpendicular to the magnetic axis **F** of the coil **120.** The magnetic channel **129** is 3D printed such that it has curved/folded path with curves/folds **127** defining between them one or more sections of the magnetic core **122** of the magnetic channel **129** which extend along the width dimension **W** of the coil **120** (i.e. traverse to the coil's magnetic axis **F).** The magnetic channel is thereby adapted to collect, via facets **126F** and **126B,** magnetic flux propagating from the direction of the magnetic axis **F** and channel the magnetic flux, via curves **127** to propagate along the core section(s) **122** thereof which extends along the longer width dimension **W** of the flattened coil **120.** In turn the windings **124** of the conductive channel **128** of the flattened coil **120** include a plurality of turns **124.1 to 124.m** that are arranged to surround the core section(s) **122** which extends along the longer width dimension **W** of the flattened coil **120.** Accordingly, the number of turns **124.1 to 124.m** that can be arranged in this manner is larger than the number of turns that could be arranged in case the core **122** was to extend in the direction along the magnetic axis **F** (i.e. along the shorted longitudinal dimension **L** of the coil **120)** and accordingly improved sensitivity of the coil to magnetic field components from the magnetic axis F is obtained, despite the flattened shape of the coil **120** in this embodiment.

In this regard it should be noted that according to some embodiments, the flux collection facets/surfaces **126F** and **126B** that face the magnetic axis **F** of the coil **120,** are made wider (e.g. with substantially larger area) than the characteristic width (e.g. cross section area) of the magnetic channel **129** between them. Even more specifically the areas of the flux collectors' facets/surfaces **126F** and **126B,** are preferably made substantially larger than the characteristic cross section area of the magnetic core section(s) **122** which are facing a different, e.g. perpendicular, direction to the magnetic axis **F.** This is in other in order for the flux collectors **126** to collect much more magnetic field flux from the direction of the magnetic axis **F** than the amount of flux that would be collected to the magnetic channel **129** from other directions, thus making flattened coil with the turned/folded magnetic channel **129** sensitive to magnetic field components directed along the magnetic axis **F,** while negligibly sensitive to magnetic field components from other directions. Optionally the flux collectors **126** and/or other sections of the magnetic channel are also tapered towards the magnetic core sections **122** (e.g. to facilitate efficient flux channeling from the relatively wide flux collection facets/surfaces **126F** and **126B** thereof to the narrower magnetic core **122).**

As indicated above, in some embodiments the 3D printing technology used for the electric component's **100** fabrication, provides for printing layers with thicknesses that are substantially smaller than the lateral widths of the printed voxels (in other words: the layer resolution is substantially higher than the resolution of in-layer pixels that can be 3D printed). For instance, in certain non-limiting examples the thicknesses of the printed layers are of about 8-10 µm (along the Z direction; typically 9µm) while the lateral sizes/widths of the printed voxels within the layers are of about 12-25 µm. Therefore, in such embodiments, 3D printing the turns **124.1 to 124.m** of the windings **124** with planar orientation in the printed layers facilitates printing of thinner turns (e.g. of about the layer thickness e.g. of 9-10 µm) and with smaller pitch of the turns, than the turn thicknesses and pitch achievable if the turn are printed with other/transverse orientation relative to the printed layers (e.g. which would result with turn thicknesses of about the lateral voxel's width, e.g. 12-25 µm, in case the turns are printed perpendicularly to and across the layers). Therefore, optionally as illustrated in **Figs. 4A and 4B****,** in some embodiments the direction **Z** of printing of the flattened coil **120** and/or the electric component **100** in which it may be contained (the direction along which the 3D printing is carried out layer by layer) is chosen/set such that the turns **124.1 to 124.m** are printed planarly within the printed layers **L₁** to **Lₙ** (for instance the printing direction **Z** of the flattened coil **120** may be set parallel to the longer width **W** dimension of the flattened coil **120** as illustrated in the figures). This, facilitates printing a stack of further more number of turns **124.1 to 124.m** surrounding the core section **122** (with higher density of turns) and thus further improving the sensitivity of the coil **120.**

It should be noted that other features of the flattened coil **120** that is exemplified in **Figs. 4A** and **4B****,** and which are not specifically described with respect to this example, may have similar properties as those discussed with reference to the example of the coils in **Figs. 1A** to **1C** and/or with in **Figs. 3A** to **3I**. For instance, optionally, in some non-limiting embodiments, functional features of the flattened coil **120,** including its conductive and magnetic channels, **128** and **129,** and/or the different parts thereof, as well as insulation **125** between conductive elements such as the turns **124.1 to 124.m,** may be 3D printed with minimal in-layer feature size of at least two voxels of the 3D printing in order to facilitate reliable 3D printing of the coil **120** that is robust to misprints of individual voxels. This may be in like manner as described above with reference to **Figs. 1A** to **1C****.** Also, the turns **124.1 to 124.m** of the windings may optionally include pluralities of concentric turns (not specifically shown), in order to further increase the count of turns surrounding the magnetic core **122,** and thus improve the coil's sensitivity. In such embodiments, optionally the spacings/insulation **125** between concentric turns may optionally be configured in like manner as described with reference to the embodiment of **Figs. 3A** to **3I****.**

In **Figs. 4A** and **4B** one optional configuration of the 3D printed flattened coil **120** (and/or of an electric component **100** containing the same) is exemplified. The magnetic channel **129** of that flattened coil **120** being curved/folded **127** such that one or more sections of the magnetic channel **129** extends substantially along the longer width dimension **W** of the flattened coil (perpendicular to its magnetic axis **F)** printing direction to serve as the magnetic core **122** of the coil and is being surrounded by conductive turns of the windings **124.** **Figs. 5A** to **8B** provide self-explanatory depictions of several other non-limiting examples of configurations of coils **120** whose magnetic channels **129** are curved/folded in order to facilitate configurations of windings to have a plurality of magnetic core sections **122** facilitating an arrangement of the winding turns and/or increasing their number improving the coils sensitivity. Like reference numerals are used in **Figs. 5A to 8B** to designate elements/sections of similar functionality as in the coils described above with reference to **Figs. 1A** to **1C** and **3A** to **4B.**

**Figs. 5A** to **5D** respectively exemplify, in self-explanatory manner, perspective-side-, front- and back- views of an electric component, in this example including a coil **120,** which may be 3D printed according to embodiments of the present invention. In this example the magnetic channel **129** is curved/folded **127** several times such that between the flux collectors **126,** the magnetic channel **129** is printed-with/has a helical-like shape with a plurality of magnetic core section **122** thereof extending substantially along the printing direction **Z.** The plurality of core sections **122** are surrounded by turns of the conductive windings **124** of the conductive channel, which are planarly printed in the 3D printed layers, with relatively small pitch/distances between them. The turns of the conductive windings **124** are typically spaced from one another by electrical insulation printed in the layers between them (e.g. the electrical insulation may be printed with between the turns with the bulk material **M3).** As shown the magnetic channel **129** is formed in a helical like shape, such that the multiple magnetic core sections **122** thereof traverse through the conductive windings **124** to channel magnetic flux, which flows between the flux collectors **126** along the magnetic channel **129,** to pass several times through the conductive windings **124** and thereby provide improved sensitivity of the coil **120.** Although in this non-limiting example the magnetic channel **129** is folded such that two magnetic core sections **122** thereof traverses through the windings **124,** it should be appreciated by those versed in the art that the coil **120** may generally be printed with other configurations of the helical channel and may be formed with any suitable number of folds **127** to yield any suitable number of magnetic core sections through the windings that provide the optimized sensitivity for the specific sensory application for which it is used.

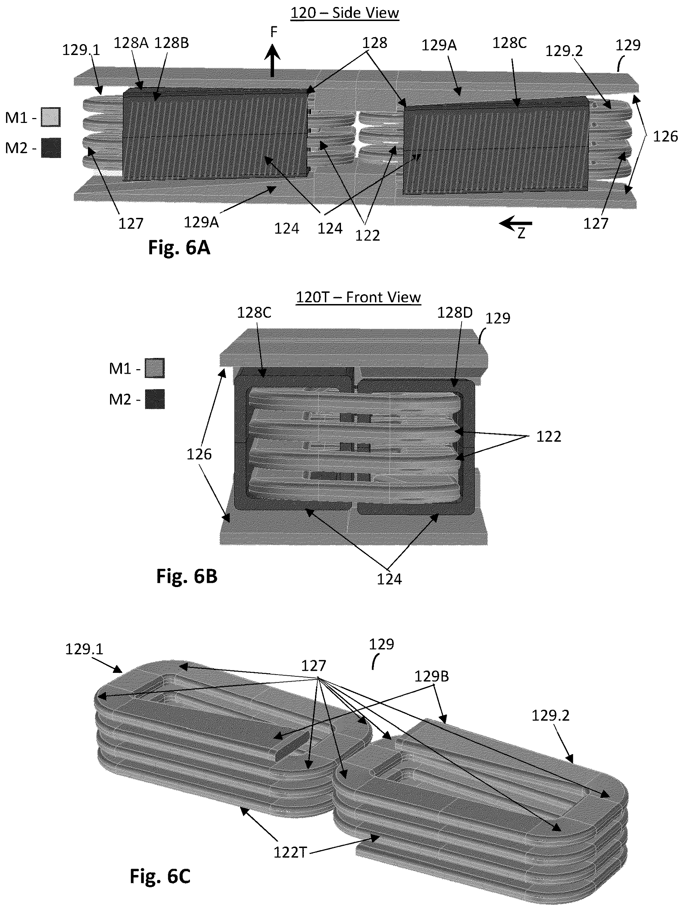

**Figs. 6A and 6C** respectively exemplify, in self-explanatory manner, side- and front views of a coil **120** with a plurality of helical magnetic sub-channels, **129.1** and **129.2,** which may be printed in the electric components **100** according to embodiments of the present invention. **Fig. 6C** is a perspective view of the helical magnetic sub-channels In this example, between the flux collectors **126,** the magnetic channel **129** is split into a plurality of magnetic subchannels (two in this example **129.1** and **129.2)** each being curved/folded **127** several times in the form of a helix (or otherwise a helical-like shape), such that the magnetic channel **129** has a multi-helix shape (double helix in this example - one helix formed by each of the magnetic subchannel **129.1** and **129.2).** The magnetic sub-channels **129.1** and **129.2** are printed with curves/folds **127** such that they define a plurality of magnetic core sections **122** extending substantially along to the printing direction **Z** and are surrounded by turns of the conductive windings **124** of the coil **120.** In this non-limiting example, the conductive windings **124** include several winding sets **128A** to **128D** each including a plurality of conductive turns surrounding different one or more of the magnetic core sections **122.** To this end, in various embodiments, the electric channel **128** between the electric contacts/terminals thereof (not specifically shown in these figures), may be 3D printed as a single conductive path passing between the pair of electric contacts/terminals and shaped to form the winding sets **128A** to **128D** along a single conductive path, or the electric channel **128** between the electric contacts may be printed to split into several (e.g. 4) electric sub channels (e.g. which may be connected to one another near the electric contacts in parallel) and each forming a plurality of windings/turns of the one or more winding sets **128A** to **128D.**

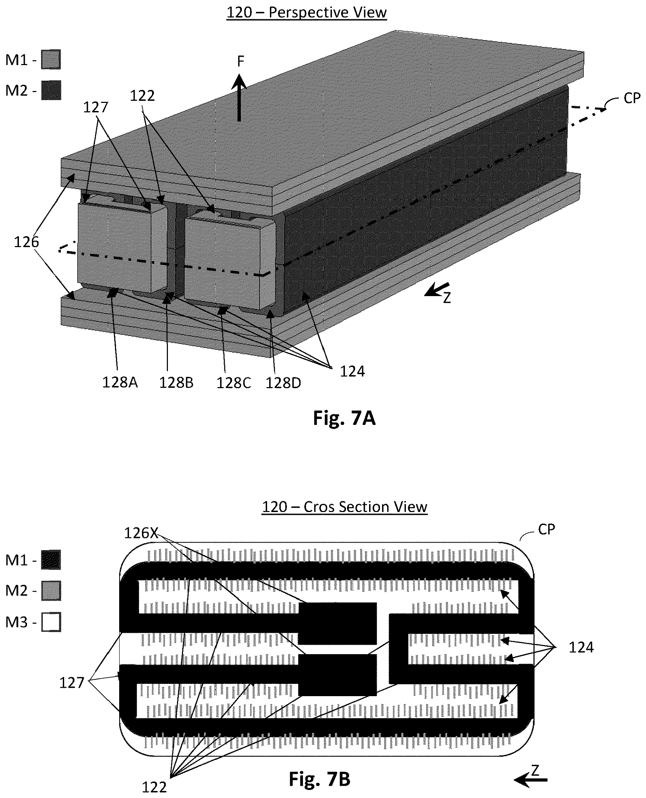

As indicated above, in certain embodiments of the present invention the magnetic channel **129** has a tapered shape/structure in a region thereof between the flux collection surfaces/facets of the flux collectors **126** and the magnetic core sections **122.** The example illustrated in **Figs. 6A to 6C** exemplifies such tapered shape of the magnetic channel **129** in which the cross-section area/width of the magnetic channel **129** is getting narrower along the direction advancing from the flax collectors **126** towards the core sections **122** of the channel. In this non-limiting example, the tapering of the magnetic channel **129** is made gradually at several location including tapering of the flux collectors **126,** as shown in the tapered regions **129A** marked in **Fig. 3A****,** as well as tapering of the section **129B** of the magnetic channel **129** that directs the magnetic flux between the flux collectors **126** to the core sections **122** as illustrated in **Fig. 6C****.** The tapering provides for improved coupling of external magnetic flux propagating collected by the flux collectors **126** to flow through the magnetic channel **126,** and thereby improves the sensitivity of the coil **120.** It should be noted that the tapering of the magnetic channel towards the magnetic core thereof, as exemplified in this embodiment in regions **129A** and/or **129B,** may also be implemented in other embodiments of the present invention, for example in coils **120** with configurations such as exemplified in **Figs. 1A** to **1C****,** **3A** to **5D** described above and/or in **Figs. 7A** to **8B** described below.

**Figs. 7A and 7B** exemplify, in self-explanatory manner, a yet another example of an electric component including a coil **120,** which may be 3D according to the present invention. **Fig. 7A** depicts a perspective view of the coil **120** and **Fig. 7B** depicts a cross-sectional view of the coil **120** taken along the plane **CP** illustrated in **Fig. 7A****.** In this example, the magnetic channel **129** extends between the flux collectors **126** in a meander-like shape/path. The flux collectors **126** are not shown **in** **Fig. 7B****,** yet instead one of the regions **126X** of the magnetic channel **129** is shown to which one of the flux collectors **126** is coupled. As in the example of **Figs. 4A** and **4B****,** also in this example the magnetic channel **129** is printed with the curves/folds **127** such that core section(s) **122** thereof (in this case a plurality of core sections) extend substantially along the printing direction **Z.** Accordingly the magnetic core sections **122** are surrounded by turns of the conductive windings **124** which include a plurality of conductive turns surrounding different one or more of the magnetic core sections **122.** To this end, in various embodiments, the electric channel **128,** may be printed as a single conductive path passing between two electric contacts/terminals (not specifically shown) at opposite ends thereof, and shaped to form the winding sets **128A** to **128D** along a single conductive path, or the electric channel **128** between the electric contacts may be printed to split into several (e.g. 4) electric sub channels forming winding sets **128A** to **128D** which may be electrically connected to one another (e.g. in series in order to increase the output voltage of the coil).

The 3D printed coils **120** with the meander-like and/or helical-like magnetic channels **129,** as exemplified in **Figs. 5A to 7B****,** may be advantageous in some cases in terms of their sensitivity. This is due to the shapes of their magnetic channel which facilitates packing longer total core sections **122** and thus higher count of windings' **124** turns surrounding them, within electric components/coils **120** having compact dimensions. In particular, coils **120** with the meander-like and/or helical-like magnetic channels **129** are suited for forming flattened coils similar to that illustrated in the embodiment of **Figs. 4A** and **4B****,** and the features of the flattened coil **120** exemplified in **Figs. 4A** and **4B** may also be implemented in the embodiments of **Figs. 5A** to **7B****.** Additionally, the windings **124,** and/or the winding sets **128A** to **128D** in the embodiments of **Figs. 5A** to **7B** may in some embodiments be implemented with concentric winding configuration including a plurality of concentrically arranged turn stacks similar to the windings **124** illustrated in **Figs. 3A** to **3I****,** in order to yield improved sensitivity of the coil **120.** In this regard, in some implementations the lateral spacing between concentrically arranged turns may be of only one voxel in a manner similar to that described with reference to **Figs. 3A** to **3I****,** in order to facilitate more compact packing of higher count of turns within compact dimensions thus further improving the coil's sensitivity.

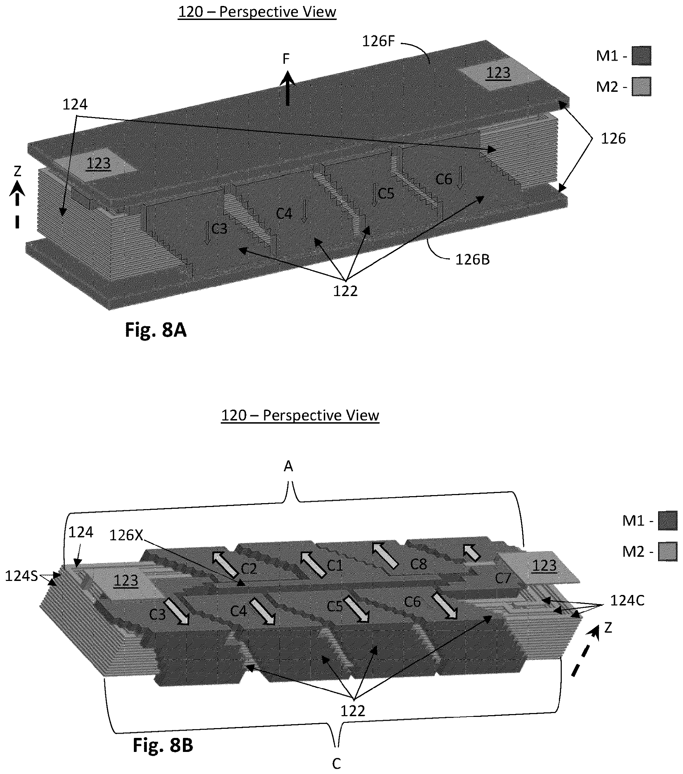

With reference to **Figs. 8A** and **8B** illustrated is a 3D printed coil **120** (e.g. or of an electric component containing it) according to an embodiment of the invention, in which the magnetic channel **129** is curved/folded to follow/form a coiled-like path/shape (e.g. such that it is wrapped about the conductive windings **124** of the coil **120).** **Fig. 8A** depicts a perspective view of the coil **120** including the flux collectors **126,** and **Fig. 5B** depicts another perspective view of the coil **120** without showing the flax collectors **126,** so that the internal configuration of the coiled magnetic core **122** and its wrapping about the conductive windings **124** is visible in the figure. Like reference numerals are used in **Figs. 8A** and **8B** to designate elements/sections/parts of similar functionality as in the coils of the other embodiments of the invention described above.

To this end, in this example the magnetic flux collected by one of the flux collectors **126** is channeled by the magnetic channel **129** to circumference the conductive turns of the windings **124,** typically for a plurality of times. For instance, in this non-limiting example, magnetic flux collected from the top flux collection surface **126U** illustrated in **Fig. 8A** is channeled through the magnetic channel **129** from the region **126X** of the magnetic channel **129** (being the connection between the flux collector to the coiled magnetic core **122)** to follow sequentially through coiled sections **C1** to **C8** of the core **122** and then exit the coil **120** though the bottom flux collection surface **126D.** The direction of the flux flow through the coiled core sections **C1** to **C8** of the magnetic core **122L** is depicted in self-explanatory manner. To this end, the coiled core sections **C1** to **C8** are wrapped/winded about the turns of the conductive windings **124** (e.g. intertwined therewith) in a manner that induces voltage/current in the conductive windings **124** in response to magnetic flux propagating through the coiled core sections **C1** to **C8,** and thereby voltage at the electric contacts/terminals **123** of the electric channel **128.**

It should be noted that the coiled core configuration illustrated in this embodiment is suitable for fabrication of a flattened coil, as described above, whereby the printing direction **Z** is not necessarily transverse to the magnetic axis **F** of the coil **120.** This is because the coiled core configuration illustrated in this example, facilitates longer core sections **122** to be winded about the windings even when the distance between the flux collectors **126** is small. Indeed, as exemplified in the optional configuration of the coil **120** illustrated in **Figs. 8A** and **8B****,** the printing direction **Z** is substantially parallel to the magnetic axis **F.** The winding turns are preferably 3D printed with planar orientations within the printing layers, as exemplified in these figures, however it should be noted that in other embodiments the winding turns may also be 3D printed in other orientations.

Alternatively, or additionally, although not illustrated in the figures, it should be understood and appreciated by those versed in the art that a coil **120** with similar coiled configuration of the magnetic core **122** may be fabricated such that its magnetic axis **F** is perpendicular to the printing direction **Z,** thus optionally implementing a flattened coil configuration similar to that described above with reference to **Figs. 4A** and **4B** but with a coiled configuration of the core **122.** For instance, in a none-limiting example, such flattened coil configuration would be obtained by arranging the flux collectors **126** to be located from the lateral sides **A** and **C** of the coil **120** which are marked in **Fig. 8B****;** or alternatively 3D printing the turns of the conductive windings **124** with orientation perpendicular to the magnetic axis (e.g. parallel to the printing direction, such the turns or some of them are printed across multiple printed layers).

It should be noted that in this non-limiting example, in order to provide further improved sensitivity of the coil **120,** the conductive windings include plurality of turns **124S** stacked along the printing direction **Z** in a plurality of printing layers (e.g. as indicated above in some embodiments printing the turns with such orientation facilitates the relatively small spacing between the stacked turns due to the layers' thicknesses being substantially smaller than the lateral width of the printed voxels). Optionally in at least some of the printing layers the multiple turns **124C** are printed concentrically in order to provide further improved sensitivity of the coil **120.** To this end in some implementations the turns of the windings **124** are printed with similar configuration and insulation between them as illustrated in the example of **Figs. 3A** to **3I** to thereby obtain compact packing of a plurality of conductive turns surrounding the core sections **C1** to **C8,** thus yielding highly sensitive coil **120** having compact dimensions.

To this end, the figures described above, schematically illustrate various non-limiting examples of 3D printed electric components **100,** and/or of 3D printed coils **120.** Preferably, in some implementations the turns of the conductive windings **124** of the coils are 3D printed planarly within the printing layers in order to exploit the smaller pitch between printing layers, which is smaller than lateral dimensions of the printed voxels, for packing/printing a higher count of conductive winding turns in the coils and facilitate high sensitivity of coils (i.e. facilitate high induced voltage in response to magnetic field sensing). Additionally, or alternatively, in some implementations, functional features of the coils/electric-components are 3D printed with minimal in-layer feature size of at least two voxels, in order to yield reliable 3D printing of the coils/electric-components. Additionally, in some implementations concentric winding turns are packed compactly with lateral spacing of only one voxel between concentrically adjacent turns. The invention also provides a technique for fabrication a high sensitivity flattened coil having a low/small aspect ratio between its length along its magnetic axis and its width traverse to its magnetic axis . This may be achieved by 3D printing at opposite ends of the magnetic channel **129,** a pair of flux collectors **126** facing the magnetic axis **F** to collect flux propagating therefrom, and further printing the magnetic channel **129** with turn(s)/fold(s)/curve(s) **127** between the pair of flux collectors so that one or more core sections **122** thereof extends along the printing direction **Z** and passes through the conductive windings **124.** Alternatively, or additionally, as indicated above a highly sensitive flattened coil may also be fabricated by utilizing a coiled core configuration. It should be understood that although the above features of the present invention are in some cases exemplified in different figures for clarity, various combinations of these features which may not have been specifically illustrated in the figure, may be implemented in coils/electric components fabricated according to the invention while without departing from the present invention.

Furthermore, it should be noted that some optional properties of the 3D printed coils, which were illustrated only in some embodiments described above, can be readily implemented in other configurations of coils **120** of the invention without departing from the invention. For instance, as would be appreciated by those versed in the art, the following optional properties/features may be implemented in various embodiments of the invention and not only in those they were depicted above:
- the splitting of the magnetic channel **129** to form a plurality of magnetic subchannels thereof between the flux collectors as illustrated in **Figs. 6A to 6C****;**
- the splitting of the electric channel to form multiple conductive paths and optionally multiple winding sets, as illustrated **in** **Figs. 3A to 3I** and **6A to 6C;** and
- the formation of a conductive windings with both stacking of turns in multiple layers as well as forming concentric turns in each, or at least some of the layers as illustrated in **Figs. 3A to 3I** and **8A to 8B****.**

With reference back to the method **200,** as indicated in **Fig. 2** optionally in some embodiments method **200** further includes operation **250** for fabricating/attaching surface mount contacts to the 3D printed electric components **100** or to the coil(s) **120,** to facilitate their installation on a circuit board via surface mount technology (SMT).

According to some embodiments of the invention, surface mounts suitable for the SMT are 3D-printed together with the 3D-printing of the components **100.** In such embodiments typically materials such as silver-palladium alloy are used for the SMT mounts' electric contacts, since the conventional materials, Tin and Nickel-Tin which are typically used in SMT mounts' electric contacts, are not suitable for the sintering process of the 3D printing technology of the invention (Tin's melting point is too low and Nickel-Tin will oxidize in the process). To this end in some embodiments the 3D-printing operation **220** of method **200** further includes 3D printing of the external/SMT contacts at respective ends of the electric contact/terminals **123** of the coils **120** or of the electric components **100.** The 3D printed SMT contacts may be for example fabricated from silver-palladium alloy (Ag-Pd) and formed with thickness of about 9µm or more to yield external Ag-Pd SMT electrodes for the 3D printed electric components **100.**

Alternatively, or additionally, in some embodiments of the invention the SMT contacts/mounts, or some of them, comprise for example chemically/electrochemically deposited Tin-plated Copper or Nickel and are fabricated/attached to the 3D printed electric components **100/coils 120** and coupled to their electric contacts **123** only after the 3D-printing and Sintering of the components **100**/coils **120.**

Typically, one or more of the coils **120** of the sensor **100,** or all of them, are designed with an open magnetic circuit configuration of their magnetic channel **129/core 122;** that is suitable for accurate sensing of magnetic fields, and in particular suitable for magnetic field bases position sensing in medical applications/devices such as in catheters. In this context, the phrase *open magnetic circuit configuration* should be understood herein as relating to coil structures with an open-ended magnetic channel that poses substantially no shielding or shunting of external magnetic fields enabling the external magnetic field pass through the coil's magnetic core.

As indicated above the 3D printed magnetic field sensor **100** of the present invention as described above, particularly when printed with the coils **120** having open magnetic circuit configurations, may be suited for use as position and/or force sensors in medical instrument/device requiring the same.

Reference is now made to **Fig. 9** illustrating a side-view of a medical instrument **1000** (e.g., probe/catheter) according to an embodiment of the present invention in which a position sensor **300** including 3D printed coils **120** configured according to the present invention, (and/or electric component(s) **100** of the invention including the same).

The medical instrument **1000** includes a housing **H** with a magnetic field-based position sensor 300 furnished therein. The medical instrument's housing **H** includes a main section **M** and a tip section **T** which is coupled at a distal end of the main section **M** via a banding coupler **J** (e.g. a spring or joint). The banding coupler **J** is configured such that the orientation of the tip section **T** can be bent relative to the longitudinal axis **LX** of the housing **H** under applied force **FC** (which may be applied for example when tip section **T** of the medical instrument **1000** is in contact with a body tissue of a patient) with banding degree corresponding to a magnitude and direction of the applied force **FC.**

In this specific non-limiting example, the position sensor **300** a location and orientation sensor **320,** and a force sensor **310,** which are implemented by 5 coils **120.1** to **120.5** one or more of which are configured and operable according to the present invention.

The location and orientation sensor **320** utilizes 3 of the coils, including coils **120.4, 120.5** and at least one of the coils **120.1** to **120.3** for measuring magnetic fields produced by one or more magnetic field sources **MF_{L}** that are located externally to the instrument **1000,** and which produce magnetic fields whose measurements are indicative of the spatial location and orientation of the medical instrument **1000.** The coils **120.4, 120.5** and the at least one coil of the coils **120.1 to 120.3** are arranged such that their magnetic axes span 3 dimensions to thereby facilitate determining the 3D location and/or orientation of the medical instrument **1000** based on measurements of the magnetic fields form the magnetic field sources **MF_{L}** by said coils.

The force sensor **310** utilizes the 3 coils **120.1** to **120.3** in order to determine the relative of orientation between the tip and the main sections, **T** and **M,** of the housing **H.** As indicated above the relative of orientation between the tip and the main sections, **T** and **M,** is indicative of the force **FC** applied by the tip section **T,** for example on a patient's tissue. To this end the force sensor **310** also includes a magnetic field source **MF_{O}** that produces a reference magnetic field whose measurement by the coils **120.1 to 120.3** provide indication of the relative orientation between the tip and main sections, **T** and **M.** The coils **120.1 to 120.3** of the orientation sensor **310** and the magnetic field source **MF_{O}** are arranged in the housing **H** at different sides of the joint **J,** so that the measurements of the magnetic field from the magnetic field source **MF_{O}** by the coils of the orientation sensor **310** provide indication of the relative orientation between the tip and the main sections, **T** and **M.** For instance, typically, the magnetic field source **MF_{O}** is arranged at the tip section **T** and the coils **120.1 to 120.3** of the orientation sensor **310** are arranged at the main section **M** with their magnetic axes parallel to the longitudinal axis **LX** of the housing **H** (although opposite arrangement may also be applicable). To this end the coils **120.1 to 120.3** are generally arranged such that their magnetic axes are collinear to one another (while not being co-planar in order to facilitate the relative orientation to be measured with respect to two angles, e.g. indicative of the pitch and yaw of the tip **T** relative to the main section).

The specific details of the technique for magnetic-field based position sensing, which is used in the present example for sensing the location and orientation of the medical instrument **1000** and/or the forced applied thereby on a patient's tissue, are generally known in the art and need not be further discussed herein in more details. For instance, U.S. patent No. U.S.8,535,308 and U.S. patent application No. U.S. 2020/015693, which are both assigned to the assignee of the present Application, disclose example configurations of catheters utilizing sensory circuits having several coils, for sensing the location and orientation of the catheter body, as well as the force applied thereby at the interface with a tissue.

In the present nonlimiting example, the magnetic-field based position sensor **300** of the medical device **1000** is implemented with one or more of the coils **120.1 to 120.5** being a 3D printed open magnetic circuit coils, which are configured according the present invention and/or fabricated by the 3D printing method described above. In this example the housing has a narrow width/diameter of about 2 mm, and accordingly the coils **120.1 to 120.5** are compact coils extending typically no more than **0.5** mm in the radial direction with respect to the longitudinal axis of the medical instrument.

In the present non-limiting example, the three coils **120.1, 120.2** and **120.3** are 3D printed coils according to the present invention, which may be implemented as an integral part of a single electric component **100** which is 3D printed utilizing the method **200** of the present invention described above. The electric component **100** in this example has a cylindrical/donut structure with a hole along its symmetry axis in the middle, and is fitted within the housing **H** of the medical instrument such that its symmetry axis is parallel to the longitudinal axis **LX** (e.g. in order to enable passage of various connection/fluid lines of the medical instrument therethrough). The two coils **120.4** and **120.5** are also 3D printed coils according to the present invention, with SMT mounts fabricated/coupled thereto according to the optional method operation **250** described above. These coils **120.4** and **120.5** are furnished on a flexible circuit board **CB** which is folded/rolled to a cylindrical shape within the housing **H** of the medical instrument **1000,** such that their magnetic axes are substantially not parallel to one another and also not parallel (typically perpendicular) to the longitudinal axis **LX** of the housing **H.** The two coils **120.4** and **120.5** are preferably similar coils having flattened geometry, and may optionally be configured according to any one of the embodiments of **Figs. 4A to 8B****,** which facilitate 3D printing of flattened coils with sufficient sensitivity for use in medical device position sensors. The conductive windings of any one or more of the coils **120.1 to 120.5** may also be implemented/configured according to the techniques described above with reference to **Figs. 3A to 3I** to yield densely packed winding turns, and thereby improved sensitivity of the coils with compact dimensions. Also, preferably, the coils are fabricated such that their functional features are printed with minimal in-layer feature size of at least to voxels of the 3D printing, as described above with reference to **Figs. 1A to 1C****.**

It should be understood that although in the present non-limiting example all of the coils **120.1 to 120.5** used in the position sensor **300** are 3D printed coils of the present invention, in other embodiments of the invention some of the coils **120.1 to 120.5** are not necessarily be 3D printed coils, for instance coils **120.4 and 120.5** may be conventional wire winded coils, without departing from the invention. Moreover although in the present non-limiting example coils **120.1 to 120.3** are printed within a single electric component **100** and coils **120.4 and 120.5** are individually 3D printed coils furnished on a circuit board (in this non-limiting example furnished with SMT technology), in other embodiments of the present invention any number or combination of the coils **120.1 to 120.5** may be either 3D printed as individual coils and/or 3D printed as parts of one or more electric components containing them which are 3D printed according to the invention. For instance, in some implementations all the 5 coils may be part of a single 3D printed electric component according to the invention.

Advantageously, 3D printing of electric components and coils of the present invention facilitates reliable, accurate and cost-effective fabrication of open and fitting of open magnetic circuit coils within the narrow dimensions of small medical devices while facilitating sufficient sensitivity of the coils for serving as accurate position sensors of such medical device.

### Examples

Example 1. A method to fabricate an electric component including a coil device. The method includes 3D printing the coil device, by:
- 3D printing a magnetic material to form a magnetic channel including a magnetic core of the coil device;
- 3D printing a conductive material to form a conductive channel, such that the conductive channel includes conductive windings of the coil device with turns surrounding the magnetic core; and
- 3D printing a non-magnetic electrically insulating material to form electrical insulation between the turns of the conductive windings of the coil device;
   wherein functional structures of the electric component, including the turns of the conductive windings of the coil device and the electrical insulation between them, are each 3D printed with minimal in-layer feature size of at least two voxels of the 3D printing, to thereby facilitate robust and reliable 3D printing of the electric component and mitigate voxel misprints.

Example 2. The method according to Example 1, wherein the 3D printing includes successive printing of a plurality of printed layers along a printing direction; and wherein the turns of the conductive windings are 3D printed planarly within the printed layers and are 3D printed in the multitude of the printed layers; and wherein the magnetic core passes through a consecutive multitude of the printed layers.

Example 3. The method according to Example 2, wherein at least some of the turns are 3D printed in consecutive layers.

Example 4. The method according to Example 2 or 3, wherein the method includes:
- 3D printing of electrical insulation between adjacent turns with minimal in-layer feature size of the electrical insulation of at least two voxels of 3D printed electrically insulating material; and
- 3D printing of conductive vias to electrically connect between turns in different layers.

Example 5. The method according to Example 4, wherein the turns of the conductive windings include adjacent concentric turns that are 3D-printed in consecutive layers of the 3D printed layers with lateral separation of one voxel between them (i.e. between the adjacent concentric turns).

Example 6. The method according to Example 5, wherein the lateral separation of the single voxel between adjacent concentric turns is arranged with respect to one or more lateral sides of the adjacent concentric turns, and the conductive vias are arranged along at least one other lateral side of the concentric turns; and wherein the lateral separation between the adjacent concentric turns along the at least one other lateral side is of at least two voxels in order to maintain electrical insulation with the minimal in-layer feature size of the at least two voxels along that at least one other lateral side.

Example 7. The method according to Example 6, wherein the turns include neighboring concentric turns 3D printed within the same layer and at least one adjacent concentric turn printed in a consecutive layer above or below the said same layer; and wherein a lateral separation between the neighboring concentric turns within the said same layer is of four voxels along the one or more lateral sides, and of six voxels along the at least one other lateral side; thereby facilitating the minimal in-layer feature size of the electrical insulation between each of the neighboring concentric turns and the at least one adjacent concentric turn.

Example 8. The method according to any one of Examples 1 to 7, wherein the magnetic channel is configured with an open magnetic circuit configuration, and the 3D printing of the magnetic channel includes printing a pair of flux collectors at opposite ends of the magnetic core having respective flux collection facets defining a magnetic axis of the coil between them.

Example 9. The method according to Example 8, wherein the flux collection facets of the flux collectors are wider than the magnetic core.

Example 10. The method according to Example 8 or 9, wherein the flux collectors are tapered with the narrower facets of their tapering connected at respective opposite ends of the magnetic core and their wider facets serving as the flux collection facets.

Example 11. The method according to any one of Examples 1 to 10, wherein the magnetic core has a rod-like shape.

Example 12. The method according to any one of Examples 8 to 11, wherein the coil is a flattened coil having a small/low aspect ratio between its length along its magnetic axis and its width traverse to its magnetic axis; and
wherein the magnetic channel is 3D printed along a curved (e.g. folded) path such that one or more sections of the magnetic core extend along a transvers direction with respect to the magnetic axis of the coils with a length of the one or more sections (e.g. the total length thereof) being larger than a distance between the pair of flux collectors; and wherein the arrangement of conductive windings of the coil includes a plurality of turns surrounding the one or more sections of the magnetic core; thereby enabling to furnish high count of the turns of the conductive windings along the length of the one or more sections of the magnetic core and obtaining improved sensitivity of the coil.

Example 13. The method according to Example 12, wherein the curved path of the magnetic channel is such that the magnetic core has a straight, rod-like shape, extending along the transvers direction with respect to the magnetic axis.

Example 14. The method according to Example 12, wherein the curved path of the magnetic channel is such that the magnetic core has a helical-like shape or meander-like shape, and wherein the turns of the conductive windings surround one or more of said sections of the helical-like shaped or meander-like shaped magnetic core.

Example 15. The method according to Example 12, wherein the curved path of the magnetic channel is such that the magnetic core has a coiled shape surrounding one or more of the turns of the conductive windings.

Example 16. The method according to any one of Examples 1 to 15, wherein the method includes 3D printing a body of the electric component with non-magnetic electrically insulating material, at regions not occupied by the magnetic and conductive channels; and wherein the conductive material of the conductive channel is fully embedded within the body so as to be isolated from environmental conditions by which it might be degraded.

Example 17. The method according to any one of Examples 1 to 16, wherein at least one of the following:
- the conductive material includes Silver;
- the conductive material includes Copper;
- the conductive material includes Palladium;
- the conductive material includes Silver-Palladium alloy;
- the magnetic material includes Ceramic-Ferrite material;
- the magnetic material includes Metal Material;
- the magnetic material is a soft magnetic material having relative permeability µᵣ in the order of 100 or more; and
- the non-magnetic dielectric material includes ceramic or glass-ceramic material.

Example 18. The method according to any one of Examples 1 to 17, wherein the 3D printing comprises:
(a) providing curable resins including:
   - magnetic material resin including particles of magnetic material suspended in curable polymer binder;
   - conductive material resin including particles of conductive material suspended in curable polymer binder;
   - non-magnetic dielectric material resin including particles of non-magnetic dielectric material suspended in curable polymer binder; and
**(b)** 3D printing the curable resins, wherein the 3D printing includes:
   - 3D printing and curing the magnetic material resin at regions of the magnetic channel;
   - 3D printing and curing the conductive material resin at regions of the conductive channel; and
   - 3D printing and curing the non-magnetic dielectric material regions of the electric component, which are not occupied by the magnetic and conductive channels;
   thereby forming a 3D printed structure of the electric component; and
(c) sintering the 3D printed structure of the electric component at temperature sufficient for burning, or driving off, polymers therefrom and thereby achieving ceramic or metal density approaching 100% in the electric component.

Example 19. An electric component fabricated by the method of any one of Examples 1 to 18.

Example 20. A flattened coil having small/low aspect ratio between its length along its magnetic axis and its width traverse to its magnetic axis; the coil includes:
a body having a bulk being 3D-printed with non-magnetic electrically insulating material;
a magnetic channel 3D printed within the body with magnetic material, wherein the magnetic channel includes: at least a pair of flux collectors having respective flux collection facets perpendicular to the magnetic axis of the coil, and a magnetic core of the coil connected between the at least two flux collectors; and
a conductive channel 3D printed in said body with electrically conductive material, wherein the conductive channel includes an arrangement of conductive windings with a plurality of turns surrounding the magnetic core; and
wherein the magnetic channel is 3D-printed with curved (e.g. folded) path such that one or more sections of the magnetic core extend along a transverse direction with respect to the magnetic axis of the coil, and the conductive channel is 3D-printed such that the turns thereof surround one or more of the sections of the magnetic core.

Example 21. An electronic component including at least one coil; the electronic component includes:
a magnetic channel including a magnetic core of the at least one coil, 3D-printed with magnetic material; and
a conductive channel 3D-printed with electrically conductive material, wherein the conductive channel includes an arrangement of conductive windings having a plurality of turns surrounding the magnetic core;
electrical insulation 3D-printed with non-magnetic electrically insulating material between the turns;
wherein 3D-printed functional structures of the coil, including the turns of the conductive windings and the electrical insulation between them, are each printed with a minimal in-layer feature size of at least two voxels of the 3D printing.

Example 22. The electronic component according to Example 21, being 3D printed by successive printing of a plurality of printed layers along a printing direction; and wherein each of the turns of the conductive windings is 3D-printed planarly within one of the printed layers and the turns are 3D printed in the multitude of the printed layers; and wherein the magnetic core passes through a consecutive multitude of the printed layers.

Example 23. The electronic component according to Example 22, wherein the electronic component includes electrical insulation 3D-printed between adjacent turns of the conductive windings with minimal in-layer feature size of the electrical insulation of at least two voxels of 3D-printed electrically insulating material; and the conductive channel includes conductive vias that electrically connect between turns in different layers.

Example 24. The electronic component according to Example 23, wherein the turns include concentrically adjacent turns 3D-printed in consecutive layers of the 3D printed layers, with lateral separation of one/single voxel between them (i.e. between the concentrically adjacent turns).

Example 25. The electronic component according to Example 24, wherein the lateral separation of the one/single voxel between concentrically adjacent turns is from one or more lateral sides of the adjacent concentric turns, and wherein the conductive vias are 3D-printed along at least one other lateral side of the concentric turns; and wherein the lateral separation between the adjacent concentric turns along the at least one other lateral side is of at least two voxels in order to maintain the minimal in-layer feature size of the insulation such that it is of at least two voxels along that at least one other lateral side.

Example 26. A method to fabricate a coil device having small/low aspect ratio between its length along its magnetic axis and its width traverse to its magnetic axis;
the method includes fabricating the coil by 3D-printing including:
   - 3D-printing of a magnetic channel including: at least a pair of flux collectors having respective flux collection facets perpendicular to the magnetic axis, and a magnetic core of the coil having a magnetic channel connected between the at least two flux collectors; and
   - 3D-printing a conductive channel including an arrangement of conductive windings with a plurality of turns surrounding the magnetic core;
wherein the magnetic channel is 3D printed with curved (e.g. folded) path such that one or more sections of the magnetic core thereof extend along a transverse direction with respect to the magnetic axis of the coil and with the turns of the conductive windings surround one or more of said sections.

Example 27. A coil device fabricated by the method of Example 26.

Example 28. A method to fabricate a coil device, the method includes:
- 3D-printing a magnetic material to form a magnetic channel including a magnetic core of the coil;
- 3D printing a conductive material to form a conductive channel including an arrangement of conductive windings with a plurality of turns surrounding the magnetic core; and
- 3D-printing a non-magnetic electrically insulating material to form electrical insulation between the turns;
   wherein 3D-printed functional structures of the coil, including the turns and the electrical insulation, are each printed with minimal in-layer feature size of at least two voxels of the 3D-printing;
   wherein the turns comprise concentrically adjacent turns 3D-printed in consecutive 3D-printed layers of the 3D-printing with lateral separation of one/single voxel between the concentrically adjacent turns.

Example 29. The method according to Example 28, wherein the lateral separation of the one/single voxel is 3D-printed relative to one or more lateral sides of the concentrically adjacent turns; wherein the 3D-printing of the conductive channel includes 3D printing of conductive vias along at least one other lateral side of the turns for electrically connecting between turns in different layers; and wherein the lateral separation between the adjacent concentric turns along the at least one other lateral side is of at least two voxels in order to maintain electrical insulation between the turns with minimal in-layer feature size at least two voxels along the at least one other lateral side.

Example 30. A coil device fabricated by the method of Examples 28 or 29.

Example 31. A method to fabricate an electric component (100) including a coil device (120), the method comprising:
characterized in that the method comprise 3D printing the coil device; wherein said 3D printing comprises:
- 3D printing a magnetic material (M1) to form a magnetic channel (129) comprising a magnetic core (122) of the coil device (120);
- 3D printing a conductive material (M2) to form a conductive channel (128), said conductive channel (128) comprising conductive windings (124) of the coil device (120) with turns surrounding the magnetic core (122); and
- 3D printing a non-magnetic electrically insulating material (M3) to form electrical insulation (125) between the turns of the conductive windings (124) of the coil device (120);
   wherein functional structures of the electric component (120), including said turns of the conductive windings (124) of the coil device (120) and said electrical insulation (125) between them, are each printed with minimal in-layer feature size of at least two voxels of said 3D printing, to thereby facilitate robust and reliable 3D printing of the electric component and mitigate voxel misprints.

Example 32. The method according to Example 31, wherein said 3D printing comprises successive printing of a plurality of printed layers (L₁ to Lₙ) along a printing direction (Z); and wherein said turns of the conductive windings (124) are 3D printed planarly within said printed layers (Lₗ to Lₙ) and are 3D printed in the multitude of the printed layers (Lₗ to Lₙ); and wherein said magnetic core (122) passes through a consecutive multitude of said printed layers (Lₗ to Lₙ).

Example 33. The method according to Example 32, wherein at least some of said turns are 3D printed in consecutive layers; and wherein the method comprises:
- 3D printing of electrical insulation 125 between adjacent turns with minimal in-layer feature size of the electrical insulation (125) of at least two voxels of 3D printed electrically insulating material (M3); and
- 3D printing of conductive vias (121) to electrically connect between turns in different layers (L₁ to Lₙ).

Example 34. The method according to Example 33, wherein said turns comprise adjacent concentric turns (124.4, 124,5) printed in consecutive layers (Lₙ₋₁, Lₙ) of the 3D printed layers (Lₗ to Lₙ) with lateral separation of one voxel between them; and wherein said lateral separation of the one voxel between adjacent concentric turns (124.4, 124,5) is arranged with respect to one or more lateral sides (B,C,D) of the adjacent concentric turns (124.4, 124,5), and said conductive vias (121) are arranged along at least one other lateral side (A) of the concentric turns (124.4, 124,5); and wherein the lateral separation (124.4-5) between the adjacent concentric turns (124.4, 124,5) along said at least one other lateral side (A) is of at least two voxels in order to maintain electrical insulation with said minimal in-layer feature size of the at least two voxels along said at least one other lateral side (A).

Example 35. The method according to Example 34, wherein said turns comprise neighboring concentric turns (124.3, 124.5) 3D printed within the same layer (Lₙ) and at least one adjacent concentric turn (124.4) printed in a consecutive layer (Lₙ₋₁) above or below said same layer (Lₙ); and wherein a lateral separation 125.3-5) between said neighboring concentric turns in said same layer (Lₙ) is of at least four voxels along said one or more lateral sides (B, C, D), and of at least six voxels along said at least one other lateral side (A); thereby facilitating said minimal in-layer feature size of the electrical insulation between the turns.

Example 36. The method according to any one of Examples 31 to 35, wherein said magnetic channel (129) is configured with an open magnetic circuit configuration; and wherein said printing of the magnetic channel (129) comprises printing a pair of flux collectors (126) at opposite ends of said magnetic core (122) having respective flux collection facets (126F,126B) defining a magnetic axis (F) of the coil (120) between them; wherein preferably one or more of the following:
- said flux collection facets (126F,126B) of the flux collectors (126) are wider than said magnetic core (122); and
- the flux collectors (126) are tapered with narrower facets of their tapering connected at respective opposite ends of the magnetic core (122) and their wider facets serving as said flux collection facets (126F,126B).

Example 37. The method according to any one of Examples 31 to 36, wherein said magnetic core (122) has a rod-like shape.

Example 38. The method according to Example 36, wherein said coil is a flattened coil having a small aspect ratio between its length (L) along its magnetic axis (F) and its width (W) traverse to its magnetic axis (F); and
wherein said magnetic channel (129) is 3D printed along a curved path such that one or more sections of the magnetic core (122) extend along a transvers direction with respect to the magnetic axis (F) of the coil (120) with a length of the one or more magnetic sections (122) being larger than a distance between said pair of flux collectors (126); and wherein said arrangement of conductive windings (124) includes a plurality of turns surrounding said one or more sections of the magnetic core (122);
thereby enabling to furnish high count of said turns along the length of the one or more sections of the magnetic core and obtaining improved sensitivity of said coil;

Example 39. The method according to Example 38, wherein said curved path of the magnetic channel (129) is configured such that at least one of the following:
- the magnetic core (122) has a straight, rod-like shape, extending along the transvers direction with respect to the magnetic axis (F);
- the magnetic core (122) has a helical-like shape or meander-like shape, and wherein said turns of the conductive windings (124) surround one or more of said sections in the helical-like shaped or meander-like shaped magnetic core (122); and
- the magnetic core (122) has a coiled shape surrounding one or more of said turns of the conductive windings (124).

Example 40. method according to any one of Examples 31 to 39, wherein at least one of the following:
- said conductive material (M2) comprises Silver;
- said conductive material (M2) comprises Copper;
- said conductive material (M2) comprises Palladium;
- said conductive material (M2) comprises Silver-Palladium alloy;
- said magnetic material (M1) comprises Ceramic-Ferrite material;
- said magnetic material (M1) comprises Metal Material;
- said magnetic material (M1) is a soft magnetic material having relative permeability µᵣ in the order of 100 or more; and
- said non-magnetic dielectric material (M3) comprises ceramic or glass-ceramic material.

Example 41. The method according to any one of Examples 31 to 40, wherein said 3D printing comprises:
**(a)** providing curable resins (210) comprising:
   - magnetic material resin comprising particles of magnetic material (M1) suspended in curable polymer binder;
   - conductive material resin comprising particles of conductive material (M2) suspended in curable polymer binder;
   - non-magnetic dielectric material resin comprising particles of non-magnetic dielectric material (M3) suspended in curable polymer binder; and
**(b)** 3D printing said curable resins (220) comprising:
   - 3D printing and curing said magnetic material resin at regions of said magnetic channel (129);
   - 3D printing and curing said conductive material resin at regions of said conductive channel (128); and
   - 3D printing and curing said non-magnetic dielectric material regions of said electric component not occupied by said magnetic and conductive channels (1298, 128);
   thereby forming a 3D printed structure of the electric component (100); and
**(c)** sintering (230) said 3D printed structure of the electric component at temperature sufficient for burning or driving off polymers therefrom and thereby achieving ceramic or metal density approaching 100% in said electric component (100).

Example 42. A coil (120) having a small aspect ratio between its length along its magnetic axis (F) and its width traverse to its magnetic axis (F) ; the coil (120) comprising:
a body (110) having a bulk 3D printed with non-magnetic electrically insulating material (M3);
a magnetic channel (129) 3D printed in said body (110) with magnetic material (M1), wherein the magnetic channel (129) comprises: at least a pair of flux collectors (126) having respective flux collection facets (126F, 126B) perpendicular to said magnetic axis (F) of the coil (120), and a magnetic core (122) of the coil (120) connected between the at least two flux collectors (126); and
a conductive channel (128) 3D printed in said body (110) with electrically conductive material (M2), wherein the conductive channel (128) comprises an arrangement of conductive windings (124) with a plurality of turns surrounding the magnetic core (122);
wherein the magnetic channel (129) is 3D printed with curves (127) such that one or more sections of the magnetic core (122) extend along a transverse direction with respect to the magnetic axis (F) of the coil (120) and wherein the conductive channel (128) is 3D printed such that said turns of the conductive windings (124) surround one or more of said sections of the magnetic core (122).

Example 43. An electronic component (100) comprising at least one coil (120); the electronic component (100) comprising:
a magnetic channel (129) comprising a magnetic core (122) of the at least one coil (120), 3D printed with magnetic material (M1); and
a conductive channel (128) 3D printed with electrically conductive material (M2), wherein the conductive channel (128) comprises an arrangement of conductive windings (124) with a plurality of turns surrounding the magnetic core (122);
electrical insulation (125) 3D printed with non-magnetic electrically insulating material (M3) between the turns;
wherein 3D printed functional structures of the coil (120), including said turns and the electrical insulation (125) between them, are each printed with minimal in-layer feature size of at least two voxels of the 3D printing.

Example 44. The electronic component according to Example 43, being 3D printed by successive printing of a plurality of printed layers (L₁ to Lₙ) along a printing direction (Z); and wherein each of said turns of the conductive windings (124) is 3D printed planarly within one of said printed layers (L₁ to Lₙ) and said turns are 3D printed in the multitude of the printed layers (Lₗ to Lₙ); and wherein said magnetic core (122) passes through a consecutive multitude of said printed layers (L₁ to Lₙ).

Example 45. The electronic component according to Example 44 comprising electrical insulation 3D printed between adjacent turns of said conductive windings with minimal in-layer feature size of the electrical insulation (125) of at least two voxels of 3D printed electrically insulating material (M3); and said conductive channel (128) comprises conductive vias (121) 3D printed to electrically connect between turns in different layers.

Example 46. The electronic component according to Example 45, wherein said turns comprise concentrically adjacent turns (124.4, 124,5) 3D printed in consecutive layers (Lₙ₋₁, Lₙ) of said 3D printed layers (Lₗ to Lₙ) with lateral separation of one voxel between them; and wherein said lateral separation (124.4-5) of the one voxel between concentrically adjacent turns (124.4, 124,5) is from one or more lateral sides (B,C,D) of the adjacent concentric turns (124.4, 124,5), and wherein said conductive vias (121) are arranged along at least one other lateral side (A) of the concentric turns; and wherein the lateral separation (124.4-5) between the adjacent concentric turns (124.4, 124,5) along said at least one other lateral side (A) is of at least two voxels such that the insulation (125) has said minimal in-layer feature size of the at least two voxels along said at least one other lateral side (A)..

## Claims

1. A coil (120) having a small aspect ratio between its length along its magnetic axis (F) and its width traverse to its magnetic axis (F) ; the coil (120) comprising:
a body (110) having a bulk 3D printed with non-magnetic electrically insulating material (M3);
a magnetic channel (129) 3D printed in said body (110) with magnetic material (M1), wherein the magnetic channel (129) comprises: at least a pair of flux collectors (126) having respective flux collection facets (126F, 126B) perpendicular to said magnetic axis (F) of the coil (120), and a magnetic core (122) of the coil (120) connected between the at least two flux collectors (126); and
a conductive channel (128) 3D printed in said body (110) with electrically conductive material (M2), wherein the conductive channel (128) comprises an arrangement of conductive windings (124) with a plurality of turns surrounding the magnetic core (122);
wherein the magnetic channel (129) is 3D printed with curves (127) such that one or more sections of the magnetic core (122) extend along a transverse direction with respect to the magnetic axis (F) of the coil (120) and wherein the conductive channel (128) is 3D printed such that said turns of the conductive windings (124) surround one or more of said sections of the magnetic core (122).

2. A method to fabricate a coil having a small aspect ratio between its length along its magnetic axis and its width traverse to its magnetic axis;
the method includes fabricating the coil by 3D-printing including:
- 3D-printing of a magnetic channel including: at least a pair of flux collectors having respective flux collection facets perpendicular to the magnetic axis, and a magnetic core of the coil having a magnetic channel connected between the at least two flux collectors; and
- 3D-printing a conductive channel including an arrangement of conductive windings with a plurality of turns surrounding the magnetic core;
wherein the magnetic channel is 3D printed with a curved path such that one or more sections of the magnetic core thereof extend along a transverse direction with respect to the magnetic axis of the coil and with the turns of the conductive windings surround one or more of said sections.

3. The method of claim 2,
further comprising 3D-printing of a bulk of a body, the bulk printed with nonmagnetic electrically insulating material;
wherein the magnetic channel is 3D printed in said body with magnetic material;
wherein the conductive channel is 3D printed in said body with electrically conductive material.

4. The coil or method of any preceding claim, wherein the magnetic channel comprises a twisted shape including a helical shape, a double helical shape, a multi-helical shape, a meander shape, and/or a coiled shape.

5. The coil or method of any preceding claim, wherein the flux collectors each comprise a surface perpendicular to the magnetic axis of the coil, and wherein each of the surfaces is larger than a cross-section area of the magnetic core, optionally wherein each of the surfaces is larger than a characteristic cross section area of the one or more sections of the magnetic core that extend along the transverse direction with respect to the magnetic axis of the coil.

6. The coil or method of any preceding claim, wherein the conductive channel comprises two ends, wherein the conductive channel is terminated at both ends by electric contacts or electric terminals.

7. The coil or method of any preceding claim, wherein the coil is configured for use in a medical catheter.

8. The coil of any of claims 1 or 4 to 7, wherein the one or more sections of the magnetic core that extend along the transverse direction with respect to the magnetic axis of the coil are defined between the curves.

9. The method of any one of claims 2 to 7, wherein the curved path comprises curves, wherein the one or more sections of the magnetic core that extend along the transverse direction with respect to the magnetic axis of the coil are defined between the curves.

10. The coil or method of any preceding claim, wherein the magnetic channel is tapered such that its cross-section area decreases along a direction advancing from the flux collectors towards the one or more sections of the magnetic core that extend along the transverse direction with respect to the magnetic axis of the coil.

11. The coil or method of any preceding claim, wherein the coil comprises 3D-printed layers of printed voxels, and wherein:
3D-printed layers each comprise a thickness of about 8-10 µm, optionally 9 µm, and wherein the printed voxels each comprise a lateral width of about 12-25 µm, and/or
wherein the plurality of turns is printed planarly within the 3D-printed layers, and/or
wherein the conductive windings comprise concentric windings in one or more of the layers.

12. The coil or method of claim 4, or of any of claims 5 to 11 when dependent on claim 4, wherein the twisted shape includes the helical shape, and wherein the magnetic channel is folded such that two or more sections thereof traverse through the conductive windings.

13. The coil or method of claim 4 or 12, or any of claims 5 to 11 when dependent on claim 4, wherein the twisted shape includes the multi-helical shape, wherein the magnetic channel is split into a plurality of magnetic subchannels between the flux collectors, wherein each magnetic subchannel has a helix or a helical-like shape.

14. The coil or method of any preceding claim, wherein the conductive windings comprise a plurality of winding sets each comprising a plurality of conductive turns surrounding one of the sections of the magnetic core that extend along the transverse direction with respect to the magnetic axis.

15. The coil or method of claim 14, wherein the winding sets are formed along a single conductive path or split to form a plurality of electrical sub channels.
